# EUROPEAN PATENT SPECIFICATION

(11) **EP 2 046 964 B1**
(45) Date of publication and mention of the grant of the patent: **04.07.2018**
(21) Application number: 07796847.7
(22) Date of filing: 11.07.2007
(51) Int. Cl.: C12N 15/85, C07H 21/04, C12P 19/34

(54) **TRANSDUCIBLE DELIVERY OF NUCLEIC ACIDS BY REVERSIBLE PHOSPHOTRIESTER CHARGE NEUTRALIZATION PROTECTING GROUPS**
TRANSDUZIERBARE ZUFÜHRUNG VON NUKLEINSÄUREN DURCH REVERSIBLE PHOSPHOTRIESTERLADUNGSNEUTRALISIERUNGS-SCHUTZGRUPPEN
DISTRIBUTION TRANSDUCTRICE D'ACIDES NUCLÉIQUES PAR DES GROUPES DE PROTECTION PHOSPHOTRIESTER RÉVERSIBLE À NEUTRALISATION DE CHARGE

(30) Priority: 12.07.2006 US 830572 P
(43) Date of publication of application: 15.04.2009
(73) Proprietor: The Regents of the University of California, Oakland, CA 94607 (US)
(72) Inventor: DOWDY, Steven, F., La Jolla, CA 92037 (US); PETERSEN, Scott, G., San Diego, CA 92124 (US); MEADE, Bryan, R., San Diego, CA 92122 (US)
(74) Representative: Harris, Jennifer Lucy
(86) International application number: PCT/US2007/015966
(87) International publication number: WO 2008/008476

(56) References cited:
- WO-A1-91/14696
- WO-A1-97/06183
- WO-A1-97/47637
- WO-A1-99/55717
- WO-A1-2006/007721
- CHAUHAN ET AL: "PTD-Fusion Peptide as a Delivery Vehicle for siRNA To Target HIV Reservoirs", MOLECULAR THERAPY, NATURE PUBLISHING GROUP, GB, vol. 13, 1 January 2006 (2006-01-01), page S277, XP005675825, ISSN: 1525-0016
- PAOELLA ET AL.: 'Electrostatic Mechanism for DNA Bending by bZIP Proteins' BIOCHEMISTRY vol. 36, 1997, pages 10033 - 10038, XP008102055
- DIAS ET AL.: 'DNA-lipid systems. A physical chemistry study' BRAZILIAN JOURNAL OF MEDICAL AND BIOLOGICAL RESEARCH vol. 35, 2002, pages 509 - 522, XP008102056

## Description

### CROSS REFERENCE TO RELATED APPLICATIONS

This application claims priority to U.S. Provisional Application Serial No. 60/830,572 filed July 12, 2006.

### TECHNICAL FIELD

This invention relates to pharmaceutical compositions compositions and pharmaceutical compositions for use in methods for transducing cells.

### BACKGROUND

Polyanionic oligomers do not readily diffuse across cell membranes. In order to overcome this problem for cultured cells, cationic lipids when combined with anionic oligonucleotides are generally used to assist uptake. Unfortunately, this complex is generally toxic to cells, which means that both the exposure time and concentration of cationic lipid must be carefully controlled to insure transfection of viable cells.

Nucleic acid delivery to cells both *in vitro* and *in vivo* has been performed using various recombinant viral vectors and electroporation. Such techniques have sought to treat various diseases and disorders by knocking-out gene expression or providing genetic constructions for gene therapy.

The discovery of RNA interference (RNAi) as a cellular mechanism that selectively degrades mRNAs allows for both the targeted manipulation of cellular phenotypes in cell culture and the potential for development of directed therapeutics (Behlke, Mol. Ther. 13, 644-670, 2006; Xie et al., Drug Discov. Today 11, 67-73, 2006). Although short interfering RNA's (siRNAs) have great potential for manipulation of cellular phenotypes, due to their size and negative (anionic) charged nature, siRNAs are macromolecules with no ability to enter cells. Indeed, siRNAs are 25x in excess of Lipinski's "Rule of 5s" for cellular delivery of membrane diffusible molecules that generally limits size to less than 500 Da. Consequently, in the absence of a delivery vehicle or transfection agent, naked siRNAs do not enter cells, even at millimolar concentrations (Barquinero et al., Gene Ther. 11 Suppl 1, S3-9, 2004). Significant attention has been focused on the use of cationic lipids that both condense the siRNA and punch holes in the cellular membrane to solve the siRNA delivery problem. Although widely used, transfection reagents fail to achieve efficient delivery into many cell types, especially primary cells and hematopoietic cell lineages (T and B cells, macrophage). Moreover, lipofection reagents often result in varying degrees of cytotoxicity ranging from mild in tumor cells to high in primary cells.

Recent cell-directed targeting approaches using antibody fusions to DNA-condensing protamine (Song et al., Nat. Biotechnol. 23, 709-717, 2005) and siRNA fusions to receptor targeted RNA aptamers (McNamara et al., Nat. Biotechnol. 24, 1005-1015, 2006) offer the potential to delivery siRNAs into select cells. WO99/55717 and WO97/47637 disclose oligonucleotides having bioreversible phosphate blocking groups.

### SUMMARY

The disclosure provides pharmaceutical compositions for delivering masked oligonucleotides or polynucleotides into living cells. Reduced anionically charged, neutral and cationically charged oligonucleotides or polynucleotides either alone or conjugated to a transduction molecule traverse cell membranes better than anionically charged nucleic acid molecules. Transiently protected oligonucleotides or polynucleotides comprising a protecting/charge neutralizing group (e.g., a cationic peptide conjugated neutral phosphotriester based oligonucleotides or polynucleotides and positively charged phosphotriester oligonucleotides or polynucleotides) can be synthesized by automated amidite methods. These compounds can enter the cytosol of living cells by endocytic or macropinocytic mechanisms. In one aspect, the phosphotriester protecting/neutralizing group when exposed to the intracellular environment is designed to be removed by enzymatic activity or by passive intracellular methods (e.g., high intracellular concentrations of glutathione) to give phosphodiester oligonucleotides or polynucleotides capable of eliciting an RNAi response. Accordingly, the disclosure provides pharmaceutical compositions containing oligonucleotide prodrugs as defined in claim 1 useful as therapeutics, diagnostics and as tools for research.

Described is a peptide RNA/DNA hybrid pro-drug where the phosphates that are normally found in a negatively charged state under physiological conditions along the backbone of an oligonucleotide or polynucleotide are transiently protected as a nonionic charge neutral phosphotriester. In one aspect, the charge neutralizing phosphotriester group is formed by the addition of any number of different phosphate protecting groups (*e.g*., S-pivaloyl thioethanol (SPTE) group). In an alternate embodiment, the phosphate backbone is protected with a biologically reversible R group that contains a positive charge, *e.g*., a guanidinium group via an enzymatically labile linker (RNB) to give net cationic character to the construct. The oligonucleotide pro-drug with a net positive charge, by way of peptide linkage or by synthetically adding positive charge to the phosphate backbone of the oligonucleotide, allows for cell membrane penetration.

Described is a nucleic acid construct comprising: a) an oligonucleotide or polynucleotide domain comprising a phosphodiester and/or phosphothioate protecting group that reduces the net anionic charge of the oligonucleotide or polynucleotide backbone; and b) a transduction domain comprising a membrane transport function operably linked to the oligonucleotide or polynucleotide domain. In one aspect, the phosphodiester and/or phosphothioate protecting group has the general formula:

R-X- or Q-X-

wherein X is O, S or NR¹, and R1 is H, methyl, ethyl, S-pivaloyl thioethanol, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic;
wherein R is selected from the group consisting of:
   R²,
      wherein R² is alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, 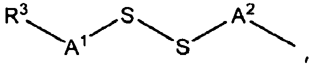
   wherein R³ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
      wherein A¹ and A² are each independently one to seven atom chains, or substituted one to seven atom chains, 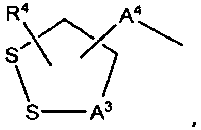
   wherein R⁴ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
      wherein A³ and A⁴ are each independently one to seven atom chains, or substituted one to seven atom chains, 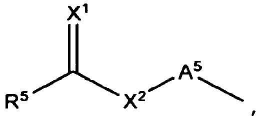
   wherein R⁵ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
      wherein A⁵ is a one to seven atom chain, or substituted one to seven atom chain,
      wherein X¹ and X² are each independently O, S or NR⁷, and R⁷ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, and 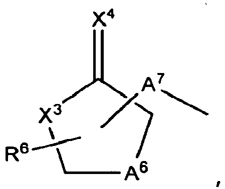
   wherein R⁶ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
      wherein A⁶ and A⁶ are each independently one to seven atom chains, or substituted one to seven atom chains,
      wherein X³ and X⁴ are each independently O, S or NR⁸, and R⁸ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic;
      wherein Q is selected from the group consisting of:
         Q¹,
            wherein Q¹ is a basic group with a pKa greater than or equal to 10, 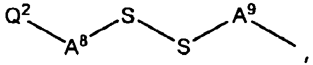
         wherein Q² is a basic group with a pKa greater than or equal to 10,
            wherein A⁸ and A⁹ are each independently one to seven atom chains, or substituted one to seven atom chains, 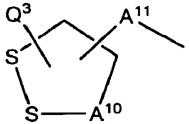
         wherein Q³ is a basic group with a pKa greater than or equal to 10,
            wherein A¹⁰ and A¹¹ are each independently one to seven atom chains, or substituted one to seven atom chains, 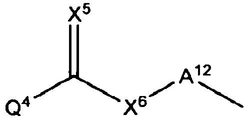
         wherein Q⁴ is a basic group with a pKa greater than or equal to 10,
            wherein A¹² is a one to seven atom chain, or substituted one to seven atom chain,
            wherein X⁵ and X⁶ are each independently O, S or NR⁹, and R⁹ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, and 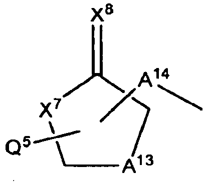
         wherein Q⁵ is a basic group with a pKa greater than or equal to 10,
            wherein A¹³ and A¹⁴ are each independently one to seven atom chains, or substituted one to seven atom chains,
               wherein X⁷ and X⁸ are each independently O, S or NR¹⁰, and R¹⁰ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic. In another aspect the phosphodiester and/or phosphothioate protecting group is selected from the group consisting of MeO-, EtO-, iPrO, 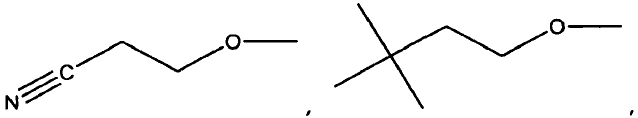 and

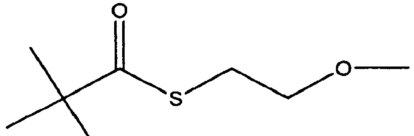

Described is a nucleic acid construct comprising: an oligonucleotide or polynucleotide comprising a phosphodiester and/or phosphothioate protecting group that reduces the net anionic charge of the oligonucleotide or polynucleotide backbone or provides a net cationic charge of the oligonucleotide or polynucleotide backbone. In one aspect, the phosphodiester and/or phosphothioate protecting group has the general formula:

R-X- or Q-X-

wherein X is O, S or NR¹, and R¹ is H, methyl, ethyl, S-pivaloyl thioethanol, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic;
wherein R is selected from the group consisting of:
   R²,
      wherein R² is alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, 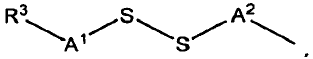
   wherein R³ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
      wherein A¹ and A² are each independently one to seven atom chains, or substituted one to seven atom chains, 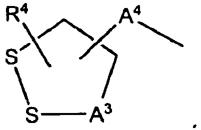
   wherein R⁴ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
      wherein A³ and A⁴ are each independently one to seven atom chains, or substituted one to seven atom chains, 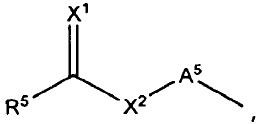
   wherein R⁵ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
      wherein A⁵ is a one to seven atom chain, or substituted one to seven atom chain,
      wherein X¹ and X² are each independently O, S or NR⁷, and R⁷ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, and 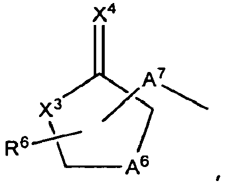
   wherein R⁶ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
      wherein A⁶ and A⁶ are each independently one to seven atom chains, or substituted one to seven atom chains,
      wherein X³ and X⁴ are each independently O, S or NR⁸, and R⁸ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic;
      wherein Q is selected from the group consisting of: Q¹,
      wherein Q¹ is a basic group with a pKa greater than or equal to 10, 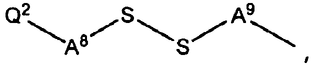
   wherein Q² is a basic group with a pKa greater than or equal to 10,
      wherein A⁸ and A⁹ are each independently one to seven atom chains, or substituted one to seven atom chains, 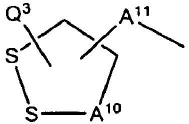
   wherein Q³ is a basic group with a pKa greater than or equal to 10,
      wherein A¹⁰ and A¹¹ are each independently one to seven atom chains, or substituted one to seven atom chains, 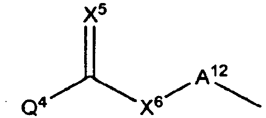
   wherein Q⁴ is a basic group with a pKa greater than or equal to 10,
      wherein A¹² is a one to seven atom chain, or substituted one to seven atom chain,
      wherein X⁵ and X⁶ are each independently O, S or NR⁹, and R⁹ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, and 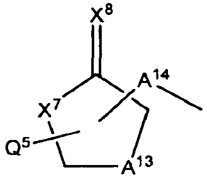
   wherein Q⁵ is a basic group with a pKa greater than or equal to 10,
      wherein A¹³ and A¹⁴ are each independently one to seven atom chains, or substituted one to seven atom chains,
         wherein X⁷ and X⁸ are each independently O, S or NR¹⁰, and R¹⁰ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic. In another aspect, the phosphodiester and/or phosphothioate protecting group is selected from the group consisting of MeO-, EtO-, iPrO, 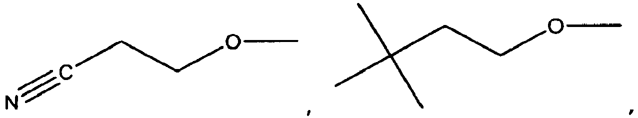 and

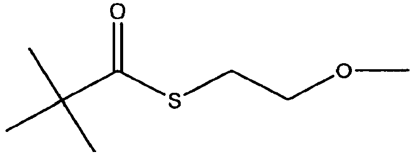

The disclosure provides pharmaceutical composition comprising the nucleic acid constructs described in claim 1.

Described is a method comprising linking one or more protein transduction domain to a nucleic acid construct. In one aspect, the one or more protein transduction domains comprise 2-5 protein transduction domains.

Described is a method of generating a nucleic acid construct comprising: substantially purifying a protein transduction domain; synthesizing an oligonucleotide; charge neutralizing the anionic charge on the oligonucleotide with a phosphotriester group; and linking the oligonucleotide to one or more protein transduction domains.

Described are methods of transfecting a cell, comprising contacting the cell with a nucleic acid construct of the disclosure. The contacting can be *in vivo* or *in vitro.* The nucleic acid construct can comprise an antisense molecule.

Described is a method of treating a disease or disorder comprising administering a nucleic acid construct of the disclosure to a subject, wherein the oligonucleotide or polynucleotide comprises a therapeutic or diagnostic molecule.

### BRIEF DESCRIPTION OF THE DRAWINGS

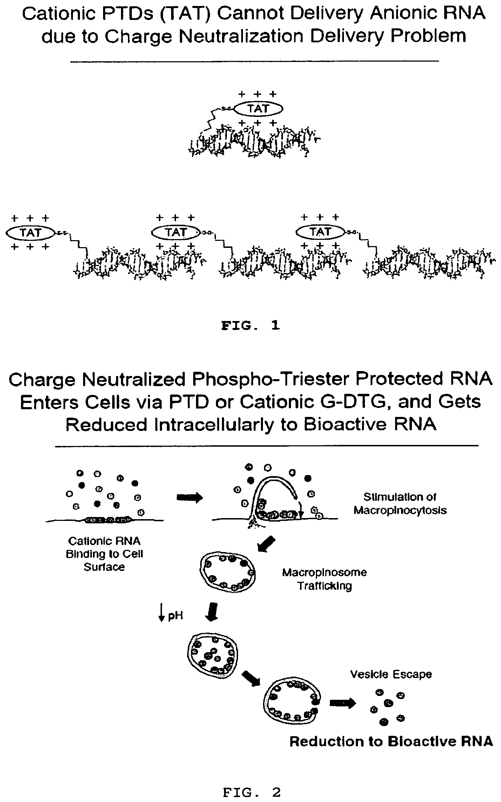

Figure 1 shows TAT and anionically charged oligonucleotide interactions. TAT linked oligonucleotides lacking a phosphodiester and/or phosphothioate protecting group form complexes wherein the cationic charges are neutralized.
Figure 2 depicts charge-neutralized oligonucleotides stimulating macropinocytosis.

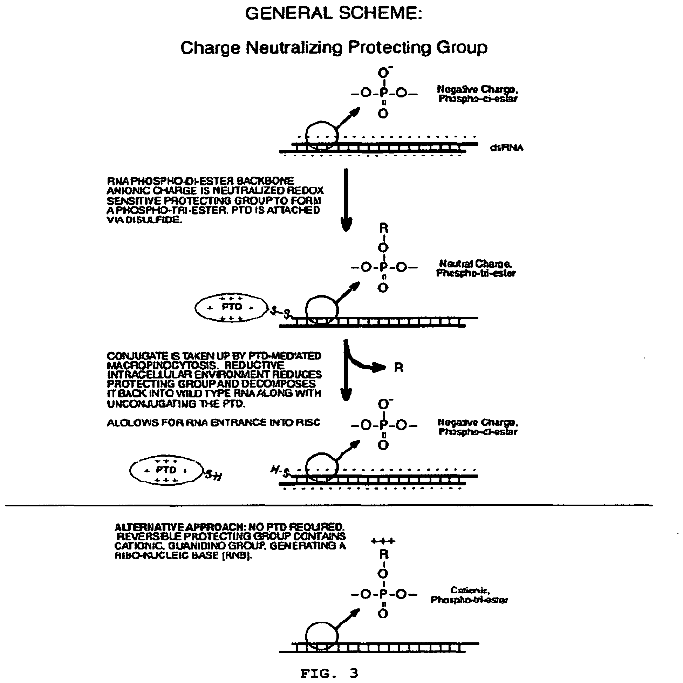

Figure 3 shows a general scheme of phosphotriester protection and charge neutralization during oligonucleotide uptake.

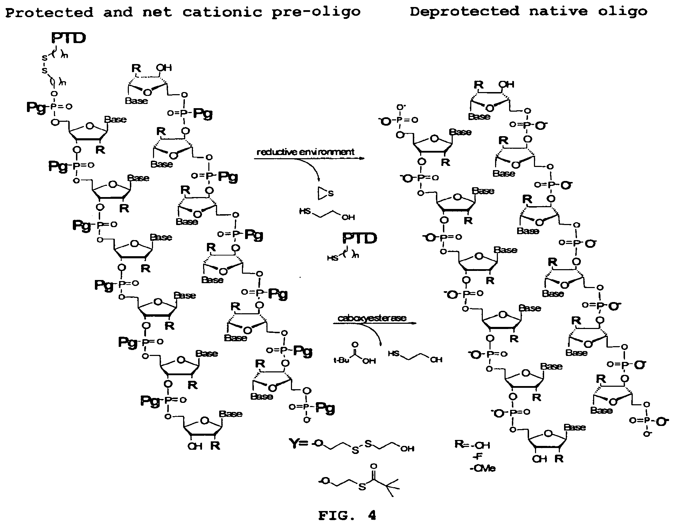

Figure 4 show an embodiment of a PTD conjugated protected oligonucleotide and reversible deprotection.

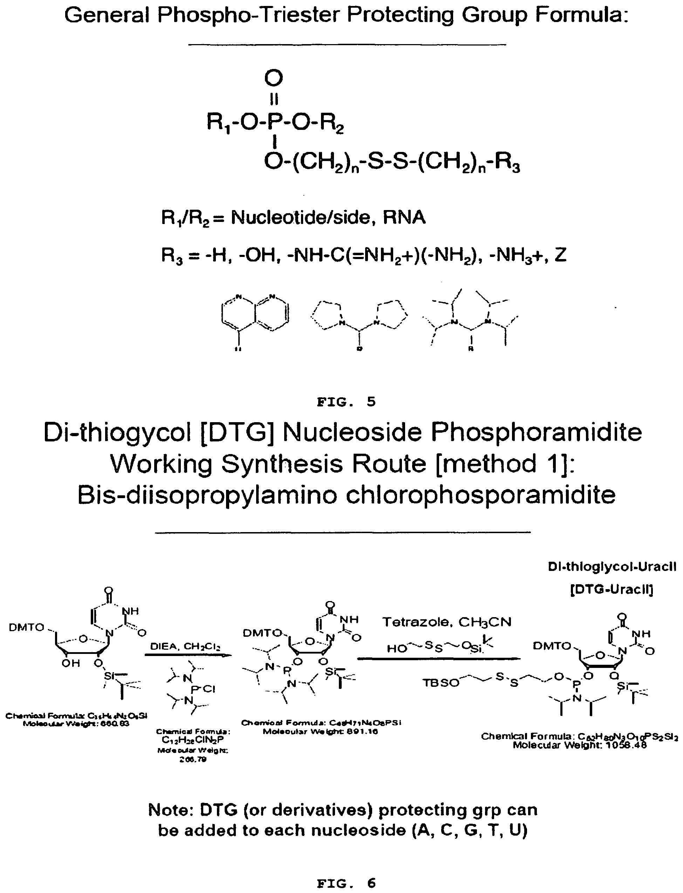

Figure 5 shows an embodiment a phosphodiester and/or phosphothioate protecting group formula.
Figure 6 shows a nucleoside phosphoramidite synthesis route of the disclosure.

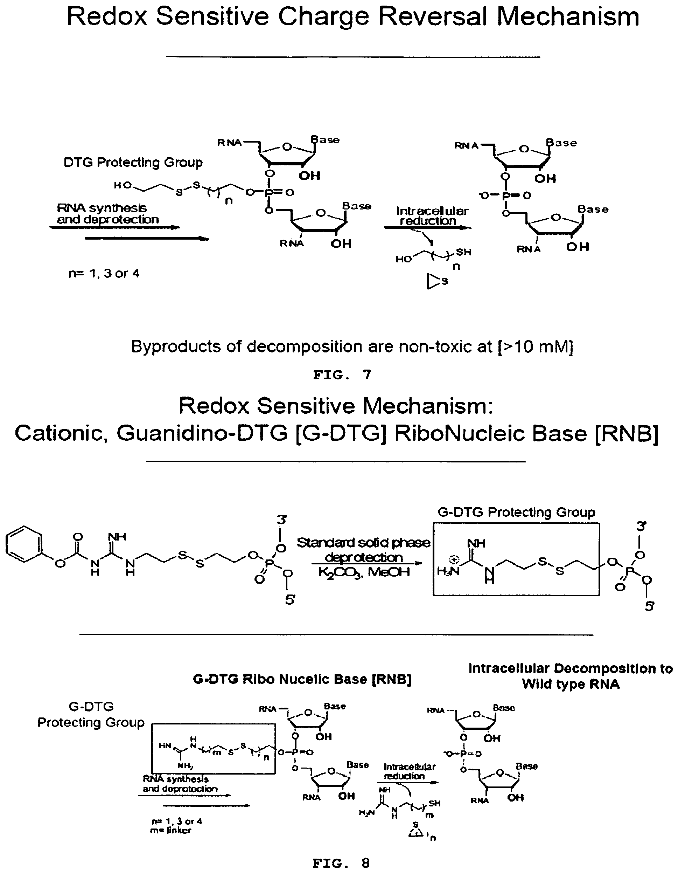

Figure 7 depicts a reduction reaction for reversible charge neutralization.
Figure 8 depicts an RNB phosphotriester protection and deprotection of anionic charged nucleic acids.

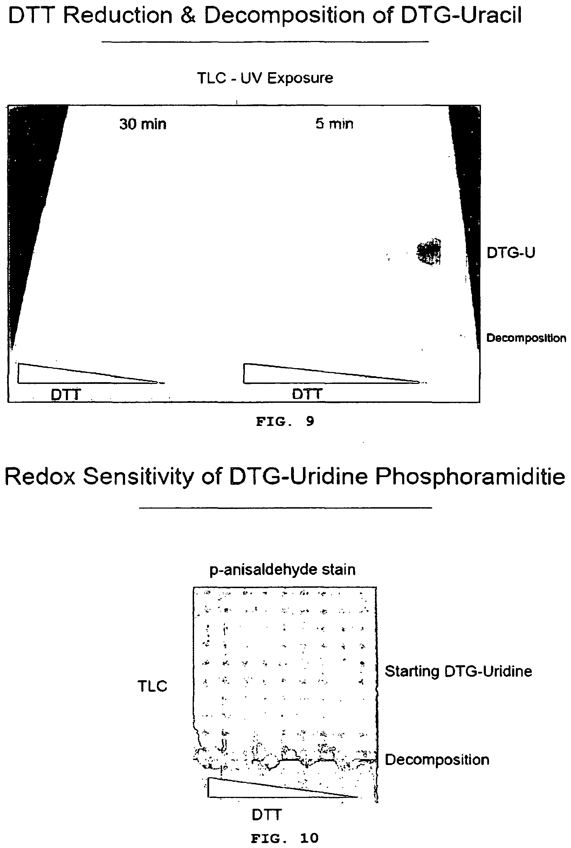

Figure 9 shows chromatographic DTT reduction of a DTG protected uracil.
Figure 10 shows redox sensitivity of DTG-Uridine phosphoramidite.

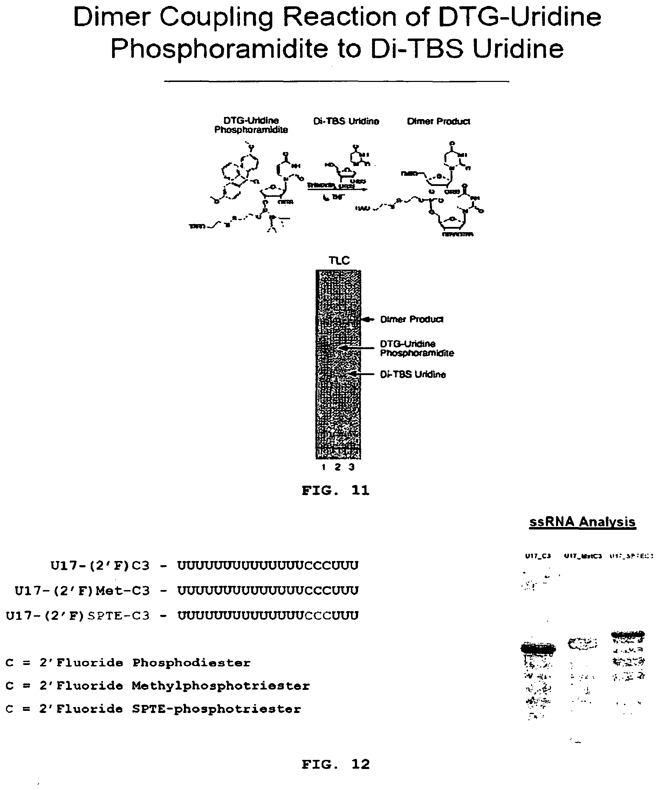

Figure 11 shows dimer coupling reaction of DTG-Uridine phosphoramidite to di-TBS uridine.
Figure 12 shows synthesis of poly-uridine oligos (SEQ ID NO:22) containing 2' fluoride insertions with wildtype phosphodiester, methylphosphotriesters or SPTE-phosphotriesters. This figure shows the ability to synthesize RNA oligonucleotides containing phosphotriesters group with a 2'-Fluoro (F). Gel analysis performed under urea denaturing conditions followed by ethidium bromide staining (right panel).

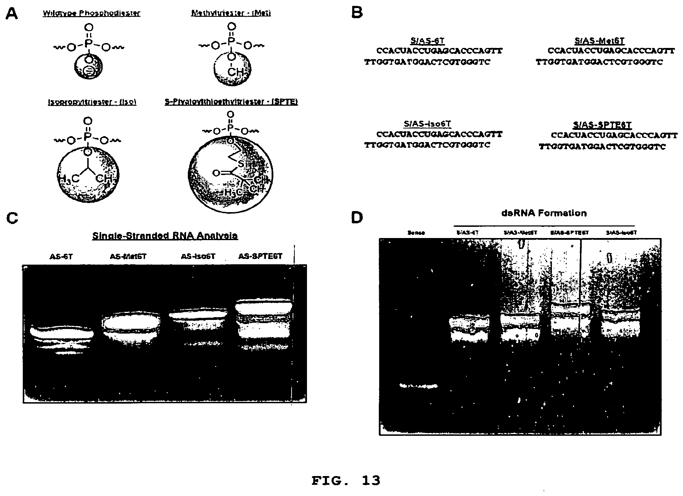

Figure 13A-D shows synthesis of double stranded siRNAs (SEQ ID NOs: 23-24) containing phosphotriester modifications. A: Structure of wild type phosphodiester and phosphotriester groups, methyl and SPTE are reversible, whereas the isopropyl group is not reversible and acts as a control. B: Sequence of eGFP siRNAs showing wild type Sense (S) strand (top line in panel) and Anti-Sense (AS) (also termed guide strand) (bottom line in panel) synthesized with six DNA thymidine insertions containing either wildtype, methyl, isopropyl or SPTE phosphotriesters as indicated. C: Single stranded RNA analysis of full length Anti-Sense (AS) oligonucleotides containing indicated phosphotriester modifications. Gel analysis performed under urea denaturing conditions followed by ethidium bromide staining. D: Double stranded RNA analysis of synthetic duplexes containing phosphotriester modifications. Sense (S) strand (top line) is wild type RNA. Anti-Sense (AS) strand (bottom line) contains 6x wild type DNA Thymidines (AS-6T), 6x methyl-phosphotriester Thymidines (AS-Met6T), 6x isopropyl-phosphotriester Thymidines (AS-Iso6T), 6x SPTE-phosphotriester Thymidines (AS-SPTE6T), as indicated. Gel analysis performed under non-denaturing conditions followed by ethidium bromide staining. This data shows the ability to make stable, full length siRNAs containing phosphotriester modifications.

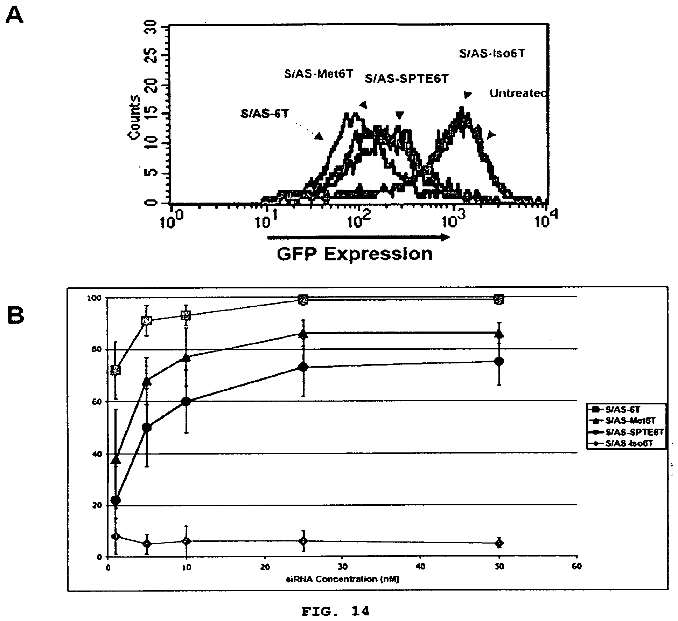

Figure 14A-B shows analysis of GFP reporter RNA Interference (RNAi) response. A: Flow cytometry (FACS) of reporter H1299 cells stably expressing eGFP following treatment with indicated siRNA duplexes. Cells were transfected with 50 nM indicated siRNAs plus Lipofectamine 2000 for 4 hr then analyzed for GFP expression by FACS at 24 hr. Untreated control cells show high level of GFP expression. Transfection of irreversible S/AS-Iso6T RNA showed no change in GFP expression, indicating a blocking or failure to induce an RNAi response by the continued presence of the 6x isopropyl phosphotriester groups. This observation demonstrates the requirement for phosphotriesters to reverse (decompose) to phosphodiesters to induce a RNAi response. Importantly, transfection of either S/AS-Met6T or S/AS-SPTE6T siRNAs induced significant RNAi responses, respectively, that were close to wild type control A/AS-6T (grey) RNAi response. These observations demonstrate the use of phosphodiesters on the Anti-Sense (guide) strand and the ability of the reversible Met6T and SPTE6T phosphotriesters groups to decompose inside the cell prior to being loaded into the RNAi machinery. B: Dose curve of indicated siRNAs from A showing RNAi efficiency at various concentrations. H1299 expressing GFP reporter cells were treated for 4 hours with siRNA plus Lipofectamine 2000. RNAi efficiency was analyzed at 24 hours post-transfection by FACS.

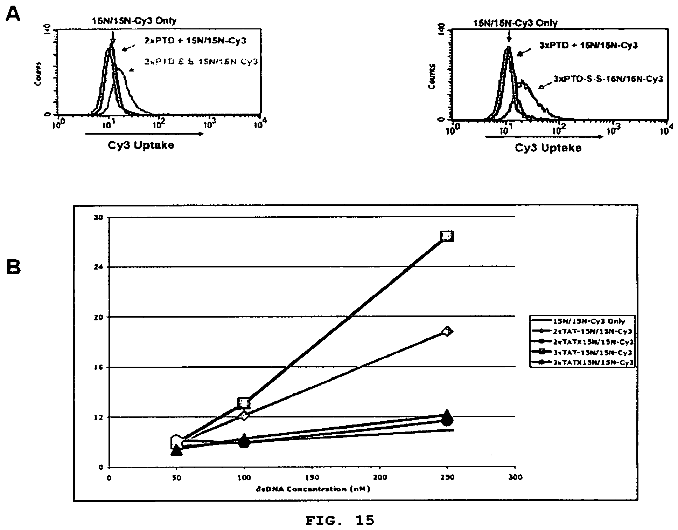

Figure 15A-B shows PTD mediated delivery of charge neutralized, dsDNA. A: dsDNA containing 15x charge neutral Methylphosphotriesters (15N) on each strand and containing a a thiol crosslinking motif on the 5' end of the Sense (S) strand and a Cy3 dye label on the 5' end of the Anti-Sense (AS) strand were were chemically cross-linked to either 2x TAT PTDs (left panel) or 3x TAT-PTDs (right panel) as indicated, purified as dsDNA molecules, added to cells for 4 hr, trypsinized, washed before analysis of siRNA uptake via Cy3 label by flow cytometry (FACS). dsDNA is only taken up when conjugated to either 2x or 3x PTD peptides via a disulfide bond (2x/3xPTD-S-S-15N/15N-Cy3), whereas control of peptide added to dsDNA, but NOT conjugated (2x/3xPTD + 15N/15N-Cy3), is not taken up by cells. B: Dose curve graph of PTD-dsDNA-Cy3 oligo conjugates from above after 4 hour treatment of H1299 cells by FACS as indicated.

### DETAILED DESCRIPTION

As used herein and in the appended claims, the singular forms "a," "and," and "the" include plural referents unless the context clearly dictates otherwise. Thus, for example, reference to "a PTD" includes a plurality of such PTDs and reference to "the cell" includes reference to one or more cells known to those skilled in the art, and so forth.

Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods and compositions, the exemplary methods, devices and materials are described herein.

The publications discussed above and throughout the text are provided solely for their disclosure prior to the filing date of the present application.

The ability to deliver certain bioactive agents to the interior of cells is problematical due to the bioavailability restriction imposed by the cell membrane. The plasma membrane of the cell forms a barrier that restricts the intracellular uptake of molecules to those which are sufficiently non-polar and smaller than approximately 500 daltons in size. Previous efforts to enhance the cellular internalization of proteins have focused on fusing proteins with receptor ligands (Ng et al., Proc. Natl. Acad. Sci. USA, 99:10706-11, 2002) or by packaging them into caged liposomal carriers (Abu-Amer et al., J. Biol. Chem. 276:30499-503, 2001). However, these techniques often result in poor cellular uptake and intracellular sequestration into the endocytic pathway.

Due to their anionic charge and large size of -14,000 Daltons, delivery of siRNA is a formidable challenge in mammals, including humans. The ability to deliver siRNA *in vivo* via protein transduction domains opens up the potential treatment of many diseases. The disclosure provides compositions to facilitate and improve cellular uptake of nucleic acid molecules by "protecting" the charge associated with an oligonucleotide or polynucleotide.

Other highly charged nucleic acid molecules with therapeutic potential face the same delivery barrier. For example, RNA aptamers have great potential to bind to, sequester and inhibit proteins, but at >10,000 Daltons and highly charged, they have no or limited ability to enter cells on their own. The compositions of the disclosure allow for intracellular delivery of RNA aptamers, siRNA and DNA vectors.

Thus, the potential of sequence specific oligonucleotides or polynucleotides to selectively treat human diseases can more effectively be delivering useful oligonucleotides and polynucleotides, including siRNAs, RNA aptamers, and DNA vectors to subjects and to cells. The disclosure overcomes size and charge limitations that make such RNAi constructs difficult to deliver or undeliverable. By reversibly neutralizing the anionic charge on a nucleic acids (*e.g*., dsRNA), a construct comprising a phosphodiester and/or phosphothioate protecting group according to the disclosure can deliver nucleic acids into a cell *in vitro* and *in vivo.*

Described are nucleic acid constructs comprising phosphodiester and/or phosphothioate protecting groups. The construct can further include compositions useful in cellular transduction and cellular modulation. Such compositions can include transduction moiety domains comprising a membrane transport function and may further comprise a nucleic acid binding domain sufficient to reversibly neutralize anionic charges on nucleic acids.

For example, charge neutralization of anionic nucleic acid (*e.g*., an RNA molecule) using a phosphodiester and/or phosphothioate protecting group(s) promotes uptake. In embodiments where the charge neutralized anionic nucleic acid is linked to a PTD the charge neutralization of the anionic charged nucleic acid frees the cationic PTD to traverse the membrane as well as prevents aggregation of the conjugate. The exposed free cationic charge of the PTD can then effectively interact with a cell surface, induce macropinocytosis and escape from the macropinosome into the cytoplasm. Once inside a cell, the phosphodiester and/or phosphothioate protecting group(s) can be removed by intracellular processes, such as reduction of a disulfide linkage or ester hydrolysis, allowing for removal from the construct in the cytoplasm. A nucleic acid construct that includes, for example, dsRNA can then be hydrolyzed by Dicer, an RNAse III-like ribonuclease, thereby releasing siRNA that silences a target gene.

The data provided herein demonstrate that the addition of one or more removable (*e.g*., reversibly attached) phosphodiester and/or phosphothioate protecting group(s) to a nucleic acid can effectively facilitate cell transduction. Any nucleic acid, regardless of sequence composition, can be modified by phosphodiester and/or phosphothioate protecting group(s).

Described are oligonucleotides or polynucleotides having, in some embodiments, one or more bioreversible protecting groups that contribute to chemical and biophysical properties that enhance cellular membrane penetration and resistance to exo- and endonuclease degradation. The disclosure further provided amidite reagents for the synthesis of the bioreversible protected oligonucleotides or polynucleotides. Moreover, these protecting groups are stable during the synthetic processes.

The oligonucleotides or polynucleotides of the disclosure having one or more bioreversible protecting groups are sometimes referred to as pro-oligonucleotides or pro-polynucleotides. In embodiments of this disclosure, the pro-oligonucleotides are capable of improved cellular lipid bilayers penetrating potential as well as resistance to exo- and endonuclease degradation *in vivo.* In cells, the bioreversible protecting groups are removed in the cell cytosol by reducing conditions, enzymatic activity (*e.g*., endogenous carboxyesterases) and the like to yield biologically active oligonucleotide compounds that are capable of hybridizing to and/or having an affinity for specific endogenous nucleic acids.

The phosphodiester and/or phosphothioate protecting groups can be used with antisense oligonucleotides of synthetic DNA or RNA or mixed molecules of complementary sequences to a target sequence belonging to a gene or to an RNA messenger whose expression they are specifically designed to block or down-regulate. The antisense oligonucleotides may be directed against a target messenger RNA sequence or, alternatively against a target DNA sequence, and hybridize to the nucleic acid to which they are complementary. Accordingly, these molecules effectively block or down-regulate gene expression.

Protected oligonucleotides or polynucleotides may also be directed against certain bicatenary DNA regions (homopurine/homopyrimidine sequences or sequences rich in purines/pyrimidines) and thus form triple helices. The formation of a triple helix, at a particular sequence, can block the interaction of protein factors which regulate or otherwise control gene expression and/or may facilitate irreversible damage to be introduced to a specific nucleic acid site if the resulting oligonucleotide is made to possess a reactive functional group.

Described herein are nucleic acid constructs, and methods of producing such constructs, that can be used for facilitating the delivery of oligonucleotides or polynucleotides in to cells. In one embodiment, a nucleic acid construct includes one or more phosphodiester and/or phosphothioate protecting group(s) to neutralize the phosphodiester anionic charge associated with a nucleic acid, such as RNA and/or DNA. Once inside the cell, the protecting group can be removed from the construct by intracellular processes that include disulfide linkage reduction, ester hydrolysis or other enzyme-mediated processes. In other embodiments, the nucleic acid construct comprising one or more phosphodiester and/or phosphothioate protecting group further comprises one or more transduction domains such as a protein transduction domain (PTD). For example, a PTD can be conjugated directly to an oligonucleotide (*e.g*., an RNA or DNA) comprising the nucleic acid construct, such as at the 5' and/or 3' end via a free thiol group. For example, a PTD can be linked to the construct by a biologically sensitive and reversible manner, such as a disulfide linkage. This approach can be applied to any oligonucleotide or polynucleotide length and will allow for delivery of RNA (*e.g*., siRNA, RNA apatmer) or DNA into cells.

In another embodiment, a nucleic acid construct can include a basic group, such as guanidium group (similar to the head group arginine, an active component of the PTD), linked to the reversible protecting group and thereby limit the need for the PTD.

Accordingly, described herein are nucleic acid constructs synthesized to include phosphodiester and/or phosphothioate protecting group(s) for the delivery of nucleic acid sequences across a cell membrane. The construct can also include, for example, one or more transduction domains and/or a protecting group that contains a basic group. Once inside the cell the oligonucleotide/polynucleotide of the nucleic acid construct reverts to an unprotected/wild type oligonucleotide/polynucleotide based on the reducing environment, by hydrolysis or other enzymatic activity.

An isolated nucleic acid construct refers to an oligonucleotide or polynucleotide associated with a molecule or compound comprising a phosphodiester and/or phosphothioate protecting group. For example, a nucleic acid construct includes, but is not limited to, an oligonucleotide or polynucleotide associated with an anionic charge reducing group(s) or molecule(s), by hydrogen bonding, charge association, covalent bonding and the like, to promote uptake by a cell. Examples of anionic charge reducing molecules that can be associated with an oligonucleotide or polynucleotide in the nucleic acid constructs of the disclosure include fusion polypeptides or peptides, chemical moieties that reduce the net anionic charge of an oligonucleotide or polynucleotide and combinations thereof.

Applications for the nucleic acid constructs provided herein include selective treatment of cancer, viral infection, genetic diseases, nucleic acid delivery for research and the like.

The term polynucleotide(s) and oligonucleotide(s) generally refers to any polyribonucleotide or polydeoxyribonucleotide, which may be unmodified RNA or DNA or modified RNA or DNA. Thus, for instance, an oligonucleotide as used herein refers to, among others, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. Thus, a oligonucleotide can comprise an siRNA, an antisense molecule, a ribozyme and the like.

In addition, a polynucleotide or oligonucleotides also includes triple-stranded regions comprising RNA or DNA or both RNA and DNA. The strands in such regions may be from the same molecule or from different molecules. The regions may include all of one or more of the molecules, but more typically involve only a region of some of the molecules.

In some aspects a polynucleotide or oligonucleotide includes DNAs or RNAs as described above that contain one or more modified bases. Thus, DNAs or RNAs with backbones comprising unusual bases, such as inosine, or modified bases, such as tritylated bases, are polynucleotides or oligonucleotides as the term is used herein.

One aspect described is a nucleic acid construct comprising an oligonucleotide with reduced anionic charged and including a phosphodiester and/or phosphothioate protecting group or charge neutralization group. However, the construct can further include transduction domains and/or nucleic acid binding domains. These approaches could include engineering a disulfide bond (or ester linkage, etc) between a nucleic acid construct (e.g., an siRNA comprising a phosphodiester protection group) and a protein transduction domain (PTD) or a PCT and a double stranded RNA binding domain (DRBD) (*e.g*., TAT-DRBD) fusion protein to further increase the binding avidity, uptake, anionic charge reduction, and/or increase cationic charge. Once taken up by a cell (*in vitro* or *in vivo*), the complex can be reduced (*e.g*., by disulfide reduction) and released inside the cell.

As used herein an anionic charge neutralizing molecule or group refers to a molecule or chemical group that can reduce the overall net anionic charge of an oligonucleotide or polynucleotide to which it is associated. Phosphodiester and/or phosphothioate protecting groups as described herein are anionic charge neutralizing groups. The phosphodiester and/or phosphothioate protecting groups can be reversible or irreversible. One or more anionic charge neutralizing molecules or groups can be associated with an oligonucleotide or polynucleotide wherein each independently contributes to a reduction or the anionic charge and or increase in cationic charge of the construct. For example, one or more phosphodiester and/or phosphothioate protecting groups can be associated with an oligonucleotide and the "protected oligonucleotide" associated with one or more cationic transduction domains (e.g., PTDs), such that the overall net anionic charge of the construct is reduced or the overall net charge of the construct is neutral or the overall net charge of the construct is cationic relative to the oligonucleotide without the phosphodiester and/or phosphothioate protecting group and/or PTD.

Described are phosphodiester and/or phosphothioate protecting groups and oligonucleotides or polynucleotides comprising such phosphodiester and/or phosphothioate protecting groups. In one embodiment, the phosphodiester and/or phosphothioate protecting group has the general formula:

R-X- or Q-X-

wherein X is O, S or NR¹, and R1 is H, methyl, ethyl, S-pivaloyl thioethanol, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic;
wherein R is selected from the group consisting of:

   R²,
wherein R² is alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic,

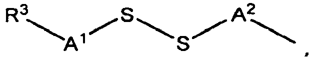

wherein R³ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
wherein A¹ and A² are each independently one to seven atom chains, or substituted one to seven atom chains,

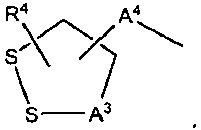

wherein R⁴ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
wherein A³ and A⁴ are each independently one to seven atom chains, or substituted one to seven atom chains,

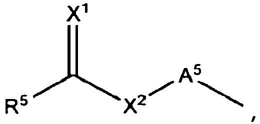

wherein R⁵ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
wherein A⁵ is a one to seven atom chain, or substituted one to seven atom chain,
wherein X¹ and X² are each independently O, S or NR⁷, and R⁷ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, and

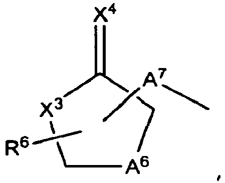

wherein R⁶ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
wherein A⁶ and A⁶ are each independently one to seven atom chains, or substituted one to seven atom chains,
wherein X³ and X⁴ are each independently O, S or NR⁸, and R⁸ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic;
wherein Q is selected from the group consisting of:
   Q¹,
wherein Q¹ is a basic group with a pKa greater than or equal to 10,

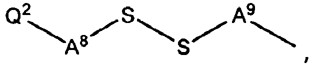

wherein Q² is a basic group with a pKa greater than or equal to 10,
wherein A⁸ and A⁹ are each independently one to seven atom chains, or substituted one to seven atom chains,

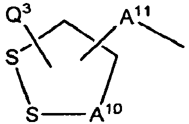

wherein Q³ is a basic group with a pKa greater than or equal to 10,
wherein A¹⁰ and A¹¹ are each independently one to seven atom chains, or substituted one to seven atom chains,

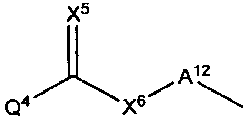

wherein Q⁴ is a basic group with a pKa greater than or equal to 10,
wherein A¹² is a one to seven atom chain, or substituted one to seven atom chain,
wherein X⁵ and X⁶ are each independently O, S or NR⁹, and R⁹ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, and

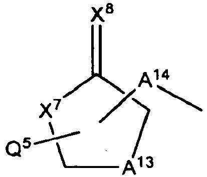

wherein Q⁵ is a basic group with a pKa greater than or equal to 10,
wherein A¹³ and A¹⁴ are each independently one to seven atom chains, or substituted one to seven atom chains,
   wherein X⁷ and x⁸ are each independently O, S or NR¹⁰, and R¹⁰ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic.

In another embodiment, the phosphodiester and/or phosphothioate protecting group is selected from the group consisting of MeO-, EtO-, iPrO, 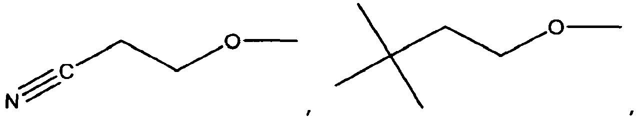 and

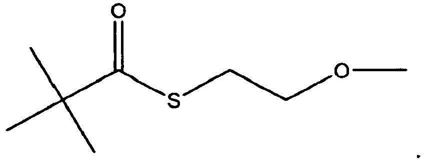

In yet another embodiment, the phosphodiester and/or phosphothioate protecting group comprises a structure selected from the group consisting of: 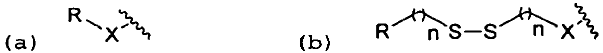 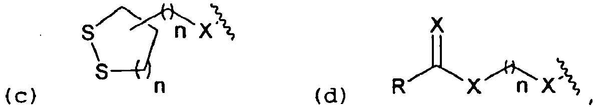 and

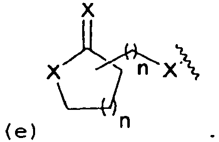

In another embodiment, the RNB protecting group comprises a structure selected from the group consisting of: 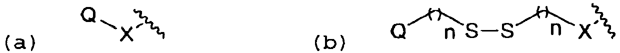 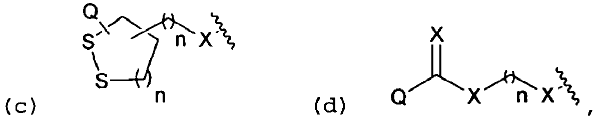 and

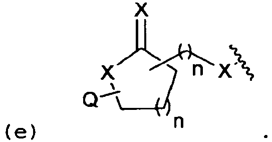

Alkyl groups include straight-chain, branched and cyclic alkyl groups. Alkyl groups include those having from 1 to 20 carbon atoms. Alkyl groups include small alkyl groups having 1 to 3 carbon atoms. Alkyl groups include medium length alkyl groups having from 4-10 carbon atoms. Alkyl groups include long alkyl groups having more than 10 carbon atoms, particularly those having 10-20 carbon atoms. Cyclic alkyl groups include those having one or more rings. Cyclic alkyl groups include those having a 3-, 4-, 5-, 6-, 7-, 8-, 9- or 1 0-member carbon ring and particularly those having a 3-, 4-, 5-, 6-, or 7-member ring. The carbon rings in cyclic alkyl groups can also carry alkyl groups. Cyclic alkyl groups can include bicyclic and tricyclic alkyl groups. Alkyl groups optionally include substituted alkyl groups. Substituted alkyl groups include among others those which are substituted with aryl groups, which in turn can be optionally substituted. Specific alkyl groups include methyl, ethyl, n-propyl, iso-propyl, cyclopropyl, n-butyl, s-butyl, t-butyl, cyclobutyl, n-pentyl, branched-pentyl, cyclopentyl, n-hexyl, branched hexyl, and cyclohexyl groups, all of which are optionally substituted.

Alkenyl groups include straight-chain, branched and cyclic alkenyl groups. Alkenyl groups include those having 1, 2 or more double bonds and those in which two or more of the double bonds are conjugated double bonds. Alkenyl groups include those having from 2 to 20 carbon atoms. Alkenyl groups include small alkyl groups having 2 to 3 carbon atoms. Alkenyl groups include medium length alkenyl groups having from 4-10 carbon atoms. Alkenyl groups include long alkenyl groups having more than 10 carbon atoms, particularly those having 10-20 carbon atoms. Cyclic alkenyl groups include those having one or more rings. Cyclic alkenyl groups include those in which a double bond is in the ring or in an alkenyl group attached to a ring. Cyclic alkenyl groups include those having a 3-, 4-, 5-, 6-, 7-, 8-, 9- or 10-member carbon ring and particularly those having a 3-, 4-, 5-, 6- or 7-member ring. The carbon rings in cyclic alkenyl groups can also carry alkyl groups. Cyclic alkenyl groups can include bicyclic and tricyclic alkyl groups. Alkenyl groups are optionally substituted. Substituted alkenyl groups include among others those which are substituted with alkyl or aryl groups, which groups in turn can be optionally substituted. Specific alkenyl groups include ethenyl, prop-1-enyl, prop-2-enyl, cycloprop-1-enyl, but-1-enyl, but-2-enyl, cyclobut-1-enyl, cyclobut-2-enyl, pent-1-enyl, pent-2-enyl, branched pentenyl, cyclopent-1-enyl, hex-1-enyl, branched hexenyl, cyclohexenyl, all of which are optionally substituted.

Aryl groups include groups having one or more 5- or 6-member aromatic or heteroaromatic rings. Aryl groups can contain one or more fused aromatic rings. Heteroaromatic rings can include one or more N, O, or S atoms in the ring. Heteroaromatic rings can include those with one, two or three N, those with one or two O, and those with one or two S. Aryl groups are optionally substituted. Substituted aryl groups include among others those which are substituted with alkyl or alkenyl groups, which groups in turn can be optionally substituted. Specific aryl groups include phenyl groups, biphenyl groups, pyridinyl groups, and naphthyl groups, all of which are optionally substituted.

Arylalkyl groups are alkyl groups substituted with one or more aryl groups wherein the alkyl groups optionally carry additional substituents and the aryl groups are optionally substituted. Specific alkylaryl groups are phenyl-substituted alkyl groups, e.g., phenylmethyl groups.

Alkylaryl groups are aryl groups substituted with one or more alkyl groups wherein the alkyl groups optionally carry additional substituents and the aryl groups are optionally substituted. Specific alkylaryl groups are alkyl-substituted phenyl groups such as methylphenyl.

The rings that may be formed from two or more of R1-R5 together can be optionally substituted cycloalkyl groups, optionally substituted cycloalkenyl groups or aromatic groups. The rings may contain 3, 4, 5, 6, 7 or more carbons. The rings may be heteroaromatic in which one, two or three carbons in the aromatic ring are replaced with N, O or S. The rings may be heteroalkyl or heteroalkenyl, in which one or more CH2 groups in the ring are replaced with O, N, NH, or S.

Optional substitution of any alkyl, alkenyl and aryl groups includes substitution with one or more of the following substituents: halogens, --CN, --COOR, --OR, --COR, --OCOOR,-CON(R) 2 , --OCON(R)2, --N(R)2, --NO2, --SR, --SO2R, --SO2N(R)2 or --SOR groups. Optional substitution of alkyl groups includes substitution with one or more alkenyl groups, aryl groups or both, wherein the alkenyl groups or aryl groups are optionally substituted. Optional substitution of alkenyl groups includes substitution with one or more alkyl groups, aryl groups, or both, wherein the alkyl groups or aryl groups are optionally substituted. Optional substitution of aryl groups includes substitution of the aryl ring with one or more alkyl groups, alkenyl groups, or both, wherein the alkyl groups or alkenyl groups are optionally substituted.

Optional substituents for alkyl, alkenyl and aryl groups include among others:
--COOR where R is a hydrogen or an alkyl group or an aryl group and more specifically where R is methyl, ethyl, propyl, butyl, or phenyl groups all of which are optionally substituted;
--COR where R is a hydrogen, or an alkyl group or an aryl groups and more specifically where R is methyl, ethyl, propyl, butyl, or phenyl groups all of which groups are optionally substituted;
--CON(R)₂ where each R, independently of each other R, is a hydrogen or an alkyl group or an aryl group and more specifically where R is methyl, ethyl, propyl, butyl, or phenyl groups all of which groups are optionally substituted; R and R can form a ring which may contain one or more double bonds;
--OCON(R)₂ where each R, independently of each other R, is a hydrogen or an alkyl group or an aryl group and more specifically where R is methyl, ethyl, propyl, butyl, or phenyl groups all of which groups are optionally substituted; R and R can form a ring which may contain one or more double bonds;
--N(R)₂ where each R, independently of each other R, is a hydrogen, or an alkyl group, acyl group or an aryl group and more specifically where R is methyl, ethyl, propyl, butyl, or phenyl or acetyl groups all of which are optionally substituted; or R and R can form a ring which may contain one or more double bonds.
--SR, --SO₂R, or --SOR where R is an alkyl group or an aryl groups and more specifically where R is methyl, ethyl, propyl, butyl, phenyl groups all of which are optionally substituted; for--SR, R can be hydrogen;
--OCOOR where R is an alkyl group or an aryl groups;
--SO₂N(R)₂ where R is a hydrogen, an alkyl group, or an aryl group and R and R can form a ring;
--OR where R=H, alkyl, aryl, or acyl; for example, R can be an acyl yielding --OCOR* where R* is a hydrogen or an alkyl group or an aryl group and more specifically where R* is methyl, ethyl, propyl, butyl, or phenyl groups all of which groups are optionally substituted.

Specific substituted alkyl groups include haloalkyl groups, particularly trihalomethyl groups and specifically trifluoromethyl groups. Specific substituted aryl groups include mono-, di-, tri, tetra- and pentahalo-substituted phenyl groups; mono-, di-, tri-, tetra-, penta-, hexa-, and hepta-halo-substituted naphthalene groups; 3- or 4-halo-substituted phenyl groups, 3- or 4-alkyl-substituted phenyl groups, 3- or 4-alkoxy-substituted phenyl groups, 3- or 4-RCO-substituted phenyl, 5- or 6-halo-substituted naphthalene groups. More specifically, substituted aryl groups include acetylphenyl groups, particularly 4-acetylphenyl groups; fluorophenyl groups, particularly 3-fluorophenyl and 4-fluorophenyl groups; chlorophenyl groups, particularly 3-chlorophenyl and 4-chlorophenyl groups; methylphenyl groups, particularly 4-methylphenyl groups, and methoxyphenyl groups, particularly 4-methoxyphenyl groups.

The use of basic containing groups (e.g., guanidium groups) linked to reversible phosphodiester and/or phosphothioate protecting groups has not been used for nucleic acid delivery. Nor have others contemplated the use of dsRNA with protecting groups and/or the use of PTDs conjugated to oligonucleotides or polynucleotides comprising a charge neutralizing group for delivery of a nucleic acid construct across a cell membrane.

Where an oligonucleotide or polynucleotide is linked to a PTD, charge neutralization of the anionically charged oligonucleotide or polynucleotide frees the cationically charged PTD to productively interact with the cell surface and also prevents aggregation of the conjugate. For example, the exposed cationic charged PTD interacts with the cell surface and induces macropinocytosis. The oligonucleotide is released into the cytoplasm. Once inside the cell, the protecting group can be cleaved off by cellular processes, such as a reducing enzyme, oxidizing enzyme, reducing agent, oxidizing agent or esterase, unprotecting the oligonucleotide or polynucleotide allowing the nucleic acid to revert to its natural configuration.

As used herein, a nucleic acid domain, used interchangeably with oligonucleotide or polynucleotide domain, can be any oligonucleotide or polynucleotide (*e.g*., a ribozyme, antisense molecule, polynucleotide, oligonucleotide and the like). Oligonucleotides or polynucleotides generally contain phosphodiester bonds, although in some cases, nucleic acid analogs are included that may have alternate backbones, comprising, *e.g*., phosphoramidate, phosphorothioate, phosphorodithioate, or O-methylphophoroamidite linkages (see Eckstein, Oligonucleotides and Analogues: A Practical Approach, Oxford University Press); and peptide nucleic acid backbones and linkages. Other analog nucleic acids include those with positive backbones; non-ionic backbones, and non-ribose backbones, including those described in U.S. Pat. Nos. 5,235,033 and 5,034,506, and Chapters 6 and 7, ASC Symposium Series 580, Carbohydrate Modifications in Antisense Research, Sanghui & Cook, eds. Nucleic acids containing one or more carbocyclic sugars are also included within one definition of nucleic acids. Modifications of the ribose-phosphate backbone may be done for a variety of reasons, *e.g*. to increase the stability and half-life of such molecules in physiological environments. Mixtures of naturally occurring nucleic acids and analogs are encompassed by the term oligonucleotide and polynucleotide; alternatively, mixtures of different nucleic acid analogs, and mixtures of naturally occurring nucleic acids and analogs can be made. Furthermore, hybrids of RNN, RNB, DNA, and RNA can be used. dsDNA, ssDNA, dsRNA, siRNA are encompassed by the term oligonucleotide and polynucleotide.

A polynucleotide refers to a polymeric compound made up of any number of covalently bonded nucleotide monomers, including nucleic acid molecules such as DNA and RNA molecules, including single- double- and triple-stranded such molecules, and is expressly intended to embrace that group of polynucleotides commonly referred to as "oligonucleotides", which are typically distinguished as having a relatively small number (no more than about 30, *e.g*., about 5-10, 10-20 or 20-30) of nucleotide constituents.

As used herein, the term "siRNA" is an abbreviation for "short interfering RNA", also sometimes known as "small interfering RNA" or "silencing RNA", and refers to a class of about 19-25 nucleotide-long double-stranded ribonucleic acid molecules that in eukaryotes are involved in the RNA interference (RNAi) pathway that results in post-transcriptional, sequence-specific gene silencing.

The term "dsRNA" is an abbreviation for "double-stranded RNA" and as used herein refers to a ribonucleic acid molecule having two complementary RNA strands and which stands distinct from siRNA in being at least about 26 nucleotides in length, and more typically is at least about 50 to about 100 nucleotides in length.

As described above, the nucleic acid may be DNA, both genomic and cDNA, RNA or a hybrid, where the nucleic acid may contain combinations of deoxyribo- and ribo-nucleotides, and combinations of bases, including uracil, adenine, thymine, cytosine, guanine, inosine, xanthine hypoxanthine, isocytosine, isoguanine, etc. As used herein, the term "nucleoside" includes nucleotides and nucleoside and nucleotide analogs, and modified nucleosides such as amino modified nucleosides. In addition, "nucleoside" includes non-naturally occurring analog structures. Thus, e.g. the individual units of a peptide nucleic acid, each containing a base, are referred to herein as a nucleoside.

The nucleic acid domain of a nucleic acid construct described herein is not limited by any particular sequence. Any number of oligonucleotide or polynucleotides useful for diagnostics, therapeutics and research can be used in the methods and compositions of the disclosure. Various sources of oligonucleotides and polynucleotides are available to one of skill in the art. For example, fragments of a genome may be isolated and the isolated polynucleotides modified in accordance with the disclosure to reduce the overall net anionic charge using phosphodiester and/or phosphothioate protecting groups or may be used as a source for extension of the oligonucleotide or polynucleotide using, for example, nucleic acid synthesis techniques known in the art.

The practice of phosphoramidite chemistry to prepare oligonucleotides is known from the published work of M. Caruthers and S. Beaucage and others. United States patents: Nos. 4,458,066, 4,500,707, 5,132,418, 4,415,732, 4,668,777, 4,973,679, 5,278,302, 5,153,319, 5,218,103, 5,268,464, 5,000,307, 5,319,079, 4,659,774, 4,672,110, 4,517,338, 4,725,677 and Re. 34,069, each of which is herein incorporated by reference, describe methods of oligonucleotide synthesis. Additionally, the practice of phosphoramidite chemistry has been systematically reviewed by Beaucage and Iyer in Beaucage, S. L. and Iyer, R. P., Tetrahedron, 1992, 48, 2223-2311 and Beaucage, S. L. and Iyer, R. P., Tetrahedron, 1993, 49, 6123-6194, or references referred to therein, all of which are herein incorporated by reference.

Nucleic acid synthesizers are commercially available and their use is generally understood by persons of ordinary skill in the art as being effective in generating nearly any oligonucleotide of reasonable length which may be desired.

In practicing phosphoramidite chemistry useful 5'OH sugar blocking groups abbreviated to DMT in the figures, are trityl, momomethoxytrityl, dimethoxytrityl and trimethoxytrityl, especially dimethoxytrityl (DMTr). In practicing phosphoramidite chemistry useful phosphite activating groups, *i.e*., NR₂, are dialkyl substituted nitrogen groups and nitrogen heterocycles. One approach includes the use of the di-isopropylamino activating group.

Various nucleoside units can be can be activated as amidites of the disclosure and incorporated in to the oligonucleotides or polynucleotides of the disclosure. These include deoxy nucleotides, *i.e*., wherein W in the above structures is H, ribonucleotides, in some embodiment W is F, *i.e*., wherein W is OH in the above structures, 2'-alkoxy nucleotides, *i.e*., wherein W is O-alkyl in the above structures, or substituted 2'-O-alkyl nucleotides, *i.e*., wherein W is substituted -O-alkyl in the above structures. 2'-O-alkyl nucleotides are described in U.S. Pat. No. 5,466,786, herein incorporated by reference. A particularly useful substituted 2'-O-alkyl group, the methoxyethoxy group, is described by Martin, P., Helv. Chim. Acta, 1995, 78, 486-504, also herein incorporated by reference.

Oligonucleotides can be synthesized by a Mermade -6 solid phase automated oligonucleotide synthesizer or any commonly available automated oligonucleotide synthesizer. Triester, phosphoramidite, or hydrogen phosphonate coupling chemistries described in, for example, M. Caruthers, Oligonucleotides: Antisense Inhibitors of Gene Expression., pp. 7-24, J. S. Cohen, ed. (CRC Press, Inc. Boca Raton, Fla., 1989) or Oligonucleotide synthesis, a practical approach, Ed. M. J. Gait, IRL Press, 1984; "Oligonucleotides and Analogues, A Practical Approach", Ed. F. Eckstein, IRL Press, 1991, are employed by these synthesizers to provide the desired oligonucleotides. The Beaucage reagent, as described in, for example, Journal of American Chemical Society, 1990, 112, 1253-1255, or elemental sulfur, as described in Beaucage et al., Tetrahedron Letters,1981, 22, 1859-1862, is used with phosphoramidite or hydrogen phosphonate chemistries to provide substituted phosphorothioate oligonucleotides. For example, the reagents comprising the protecting groups recited herein can be used in numerous applications where protection is desired. Such applications include, but are not limited to, both solid phase and solution phase, oligo- synthesis, polynucleotide synthesis and the like. The use of nucleoside and nucleotide analogs is also contemplated by this disclosure to provide oligonucleotide or oligonucleoside analogs bearing the protecting groups disclosed herein. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into an oligonucleotide or oligonucleoside sequence, they allow hybridization with a naturally occurring oligonucleotide sequence in solution. Typically, these analogs are derived from naturally occurring nucleosides and nucleotides by replacing and/or modifying the base, the ribose or the phosphodiester moiety. The changes can be tailor made to stabilize or destabilize hybrid formation or enhance the specificity of hybridization with a complementary nucleic acid sequence as desired.

For instance, structural groups are optionally added to the ribose or base of a nucleoside for incorporation into an oligonucleotide, such as a methyl, propyl or allyl group at the 2'-0 position on the ribose, or a fluoro group which substitutes for the 2'-O group, or a bromo group on the ribonucleoside base. For use with phosphoramidite chemistry, various amidite reagents are commercially available, including 2'-deoxy amidites, 2'-O-methyl amidites and 2'-O-hydroxyl amidites. Any other means for such synthesis may also be employed. The actual synthesis of the oligonucleotides is well within the talents of those skilled in the art. It is also well known to use similar techniques to prepare other oligonucleotides such as the phosphorothioates, methyl phosphonates and alkylated derivatives. It is also well known to use similar techniques and commercially available modified amidites and controlled-pore glass (CPG) products such as biotin, Cy3, fluorescein, acridine or psoralen-modified amidites and/or CPG (available from Glen Research, Sterling Va.) to synthesize fluorescently labeled, biotinylated or other conjugated oligonucleotides.

Although the phosphotriester neutralizing/protecting groups described herein are useful for neutralizing the anionic charge of a nucleic acid domain, additional cationically charged moieties linked to a protected nucleic acid domain can be used to further facilitate uptake of oligonucleotide and polynucleotides. The recent discovery of several proteins which could efficiently pass through the plasma membrane of eukaryotic cells has led to the identification of a novel class of proteins from which peptide transduction domains have been derived.

A number of protein transduction domains/peptides are known in the art and have been demonstrated to facilitate uptake of heterologous molecules linked to the transdomain (*e.g.*, cargo molecules). Such transduction domains facilitate uptake through a process referred to as macropinocytosis. Macropinocytosis is a nonselective form of endocytosis that all cells perform.

The best characterized of these proteins are the Drosophila homeoprotein antennapedia transcription protein (AntHD) (Joliot et al., New Biol. 3:1121-34, 1991; Joliot et al., Proc. Natl. Acad. Sci. USA, 88:1864-8, 1991; Le Roux et al., Proc. Natl. Acad. Sci. USA, 90:9120-4, 1993), the herpes simplex virus structural protein VP22 (Elliott and O'Hare, Cell 88:223-33, 1997), the HIV-1 transcriptional activator TAT protein (Green and Loewenstein, Cell 55:1179-1188, 1988; Frankel and Pabo, Cell 55:1189-1193, 1988), and more recently the cationic N-terminal domain of prion proteins. Not only can these proteins pass through the plasma membrane but the attachment of other proteins, such as the enzyme β-galactosidase, was sufficient to stimulate the cellular uptake of these complexes. Such chimeric proteins are present in a biologically active form within the cytoplasm and nucleus. Characterization of this process has shown that the uptake of these fusion polypeptides is rapid, often occurring within minutes, in a receptor independent fashion. Moreover, the transduction of these proteins does not appear to be affected by cell type and can efficiently transduce 100% of cells in culture with no apparent toxicity (Nagahara et al., Nat. Med. 4:1449-52, 1998). In addition to full-length proteins, protein transduction domains have also been used successfully to induce the intracellular uptake of DNA (Abu-Amer, supra), antisense oligonucleotides (Astriab-Fisher et al., Pharm. Res, 19:744-54, 2002), small molecules (Polyakov et al., Bioconjug. Chem. 11:762-71, 2000) and even inorganic 40 nanometer iron particles (Dodd et al., J. Immunol. Methods 256:89-105, 2001; Wunderbaldinger et al., Bioconjug. Chem. 13:264-8, 2002; Lewin et al., Nat. Biotechnol. 18:410-4, 2000; Josephson et al., Bioconjug., Chem. 10:186-91, 1999) suggesting that there is no apparent size restriction to this process. The effective transduction using transduction domains is, in part, limited by the overall molecular charge on the PTD-cargo construct.

The fusion of a protein transduction domain (PTD) with a heterologous molecule (*e.g*., a polynucleotide, small molecule, or protein) is sufficient to cause their transduction into a variety of different cells in a concentration-dependent manner. Moreover, this technique for protein delivery appears to circumvent many problems associated with DNA and drug based techniques.

PTDs are typically cationic in nature. These cationic protein transduction domains track into lipid raft endosomes carrying with them their linked cargo and release their cargo into the cytoplasm by disruption of the endosomal vesicle. Examples of PTDs include AntHD, TAT, VP22, cationic prion protein domains, poly-Arg, AGRKKRRQRRR (SEQ ID NO:15), YARKARRQARR (SEQ ID NO:16), YARAAARQARA (SEQ ID NO:17), YARAARRAARR (SEQ ID NO:18), YARAARRAARA (SEQ ID NO:19), YARRRRRRRRR (SEQ ID NO:20), YAAARRRRRRR (SEQ ID NO:21) and functional fragments and variants thereof. The disclosure provides, in one aspect, methods and compositions that combine the use of PTDs such as TAT and poly-Arg, with a charge neutralized nucleic acids. By charge neutralized is meant that the anionic charge of the nucleic acid (e.g., oligonucleotide or polynucleotide) is reduced, neutralized or more cationic than the same nucleic acid in the absence of a phosphodiester and/or phosphothioate protecting group or a phosphodiester and/or phosphothioate protecting group and a binding domain capable of neutralizing the anionic charge on a nucleic acid (*i.e*., the "cargo") domain.

In general, the transduction domain of a nucleic acid construct of the disclosure can be nearly any synthetic or naturally-occurring amino acid sequence that can transduce or assist in the transduction of the fusion molecule. Typically, the transduction domain is cationically charged. For example, transduction can be achieved in accord with the disclosure by use of a nucleic acid construct including phosphodiester and/or phosphothioate protecting groups and a protein sequence such as an HIV TAT protein or fragment thereof that is linked at the N-terminal or C-terminal end to an oligonucleotide or polynucleotide comprising a phosphodiester and/or phosphothioate protecting group. In some aspects, the nucleic acid may comprise a phosphodiester and/or phosphothioate protecting group and may also comprise a nucleic acid binding domain (*e.g*., a DRBD). The transducing protein domain, for example, can be the Antennapedia homeodomain or the HSV VP22 sequence, the N-terminal fragment of a prion protein or suitable transducing fragments thereof such as those known in the art.

The type and size of the PTD will be guided by several parameters including the extent of transduction desired. PTDs will be capable of transducing at least about 20%, 25%, 50%, 75%, 80%, 90%, 95%, 98% 99% or 100% of the cells. Transduction efficiency, typically expressed as the percentage of transduced cells, can be determined by several conventional methods.

PTDs will manifest cell entry and exit rates (sometimes referred to as *k₁* and *k₂*, respectively) that favor at least picomolar amounts of the fusion molecule in the cell. The entry and exit rates of the PTD and any cargo can be readily determined, or at least approximated, by standard kinetic analysis using detectably-labeled fusion molecules. Typically, the ratio of the entry rate to the exit rate will be in the range of between about 5 to about 100 up to about 1000.

In one aspect, a PTD useful in the methods and compositions of the disclosure comprise a peptide featuring substantial alpha-helicity. It has been discovered that transduction is optimized when the PTD exhibits significant alpha-helicity. In another embodiment, the PTD comprises a sequence containing basic amino acid residues that are substantially aligned along at least one face of the peptide. A PTD domain of the disclosure may be a naturally occurring peptide or a synthetic peptide.

In one embodiment of the disclosure, the PTD comprises an amino acid sequences comprising a strong alpha helical structure with arginine (Arg) residues down the helical cylinder. In yet another embodiment, the PTD domain comprises a peptide represented by the following general formula: B₁-X₁-X₂-X₃-B₂-X₄-X₅-B₃ (SEQ ID NO:1) wherein B₁, B₂, and B₃ are each independently a basic amino acid, the same or different; and X₁, X₂, X₃, X₄ and X₅ are each independently an alpha-helix enhancing amino acid, the same or different. In another embodiment, the PTD domain is represented by the following general formula: B₁-X₁-X₂-B₂-B₃-X₃-X₄-B₄ (SEQ ID NO:2) wherein B₁, B₂, B₃, and B₄ are each independently a basic amino acid, the same or different; and X₁, X₂, X₃, and X₄ are each independently an alpha-helix enhancing amino acid the same or different.

Additionally PTD domains comprise basic residues, *e.g.,* lysine (Lys) or arginine (Arg), and further including at least one proline (Pro) residue sufficient to introduce "kinks" into the domain. Examples of such domains include the transduction domains of prions. For example, such a peptide comprises KKRPKPG (SEQ ID NO:3).

In one embodiment, the domain is a peptide represented by the following sequence: X-X-R-X-(P/X)-(B/X)-B-(P/X)-X-B-(B/X) (SEQ ID NO:4), wherein X is any alpha helical promoting residue such as alanine; P/X is either proline or X as previously defined; B is a basic amino acid residue, *e.g.,* arginine (Arg) or lysine (Lys); R is arginine (Arg) and B/X is either B or X as defined above.

In another embodiment the PTD is cationic and consists of between 7 and 10 amino acids and has the formula K-X₁-R-X₂-X₁ (SEQ ID NO:5) wherein X₁ is R or K and X₂ is any amino acid. An example of such a peptide comprises RKKRRQRRR (SEQ ID NO:6).

Additional transducing domains include a TAT fragment that comprises at least amino acids 49 to 56 of TAT up to about the full-length TAT sequence (see, e.g., SEQ ID NO:7). A TAT fragment may include one or more amino acid changes sufficient to increase the alpha-helicity of the fragment. In some instances, the amino acid changes introduced will involve adding a recognized alpha-helix enhancing amino acid. Alternatively, the amino acid changes will involve removing one or more amino acids from the TAT fragment that impede alpha helix formation or stability. In a more specific embodiment, the TAT fragment will include at least one amino acid substitution with an alpha-helix enhancing amino acid. Typically a TAT fragment or other PTD will be made by standard peptide synthesis techniques although recombinant DNA approaches may be used in some cases.

Additional transduction proteins (PTDs) that can be used in the nucleic acid constructs of the disclosure include the TAT fragment in which the TAT 49-56 sequence has been modified so that at least two basic amino acids in the sequence are substantially aligned along at least one face of the TAT fragment. Illustrative TAT fragments include at least one specified amino acid substitution in at least amino acids 49-56 of TAT which substitution aligns the basic amino acid residues of the 49-56 sequence along at least one face of the segment and typically the TAT 49-56 sequence.

Additional transduction proteins include the TAT fragment in which the TAT 49-56 sequence includes at least one substitution with an alpha-helix enhancing amino acid. In one embodiment, the substitution is selected so that at least two basic amino acid residues in the TAT fragment are substantially aligned along at least one face of that TAT fragment. In a more specific embodiment, the substitution is chosen so that at least two basic amino acid residues in the TAT 49-56 sequence are substantially aligned along at least one face of that sequence.

Also included are chimeric PTD domains. Such chimeric transducing proteins include parts of at least two different transducing proteins. For example, chimeric transducing proteins can be formed by fusing two different TAT fragments, e.g., one from HIV-1 and the other from HIV-2 or one from a prion protein and one from HIV.

PTDs can be linked or fused with any number of other molecules including an oligonucleotide or polynucleotide. Alternatively, the nucleic acid construct or PTD can be bound to other molecular entities including nucleic acid binding domains, targeting moieties and the like. For example, two or more PTDs (*e.g.,* 1-5, 2-4, typically 3) can be linked in series or separated by one or more other domains (*e.g.,* a nucleic acid domain or peptide linkers). A nucleic acid binding domain can promote uptake of a fusion construct comprising a nucleic acid (including an oligonucleotide or polynucleotide comprising a protecting group) by reducing the anionic charge such that the cationic charge of the PTD domain is sufficient to transduce/traverse a cell's membrane. It will be understood that the PTD may be fused to an oligonucleotide or polynucleotide comprising a phosphodiester and/or phosphothioate protecting group and may further be linked to a nucleic acid binding domain. Exemplary RNA binding proteins (*e.g.,* DRBD) include histone, RDE-4 protein, or protamine. Additional dsRNA binding proteins (and their Accession numbers in parenthesis) include: PKR (AAA36409, AAA61926, Q03963), TRBP (P97473, AAA36765), PACT (AAC25672, AAA49947, NP609646), Staufen (AAD17531, AAF98119, AAD17529, P25159), NFAR1 (AF167569), NFAR2 (AF167570, AAF31446, AAC71052, AAA19960, AAA19961, AAG22859), SPNR (AAK20832, AAF59924, A57284), RHA (CAA71668, AAC05725, AAF57297), NREBP (AAK07692, AAF23120, AAF54409, T33856), kanadaptin (AAK29177, AAB88191, AAF55582, NP499172, NP198700, BAB19354), HYL1 (NP563850), hyponastic leaves (CAC05659, BAB00641), ADAR1 (AAB97118, P55266, AAK16102, AAB51687, AF051275), ADAR2 P78563, P51400, AAK17102, AAF63702), ADAR3 (AAF78094, AAB41862, AAF76894), TENR (XP059592, CAA59168), RNaseIII (AAF80558, AAF59169, Z81070Q02555/S55784, PO5797), and Dicer (BAA78691, AF408401, AAF56056, S44849, AAF03534, Q9884), RDE-4 (AY071926), FLJ20399 (NP060273, BAB26260), CG1434 (AAF48360, EAA12065, CAA21662), CG13139 (XP059208, XP143416, XP110450, AAF52926, EEA14824), DGCRK6 (BAB83032, XP110167) CG1800 (AAF57175, EAA08039), FLJ20036 (AAH22270, XP134159), MRP-L45 (BAB14234, XP129893), CG2109 (AAF52025), CG12493 (NP647927), CG10630 (AAF50777), CG17686 (AAD50502), T22A3.5 (CAB03384) and Accession number EAA14308.

As noted, two or more components of the constructs disclosed herein, *e.g.*, a PTD, a nucleic acid binding domain, an oligonucleotide/polynucleotide domain, and peptide linkers, can be organized in nearly any fashion provided that the construct has the function for which it was intended (*e.g*., sufficiently cationic or having reduced anionic charge). The constructs can include fusion polypeptides or chimeric proteins comprising one or more PTDs linked either directly or indirectly linked to a oligonucleotide or polynucleotide domain (*e.g*., a therapeutic or diagnostic DNA, RNA, siRNA and the like). Each of the several domains may be directly linked or may be separated by a linker peptide. The domains may be presented in any order. Additionally, the fusion polypeptides may include tags, *e.g.,* to facilitate identification and/or purification of the fusion polypeptide, such as a 6xHIS tag.

Peptide linkers that can be used in the fusion polypeptides and methods of the disclosure will typically comprise up to about 20 or 30 amino acids, commonly up to about 10 or 15 amino acids, and still more often from about 1 to 5 amino acids. The linker sequence is generally flexible so as not to hold the fusion molecule in a single rigid conformation. The linker sequence can be used, *e.g.,* to space the PTD domain from the nucleic acid binding domain and/or nucleic acid domain. For example, the peptide linker sequence can be positioned between the protein transduction domain and the nucleic acid domain, *e.g*., to provide molecular flexibility. The length of the linker moiety is chosen to optimize the biological activity of the polypeptide comprising a PTD domain fusion construct and can be determined empirically without undue experimentation. The linker moiety should be long enough and flexible enough to allow a nucleic acid binding domain to freely interact with a nucleic acid or vice versa. Examples of linker moieties are --Gly--Gly--, GGGGS (SEQ ID NO:8), (GGGGS)N (SEQ ID NO:9), GKSSGSGSESKS (SEQ ID NO:10), GSTSGSGKSSEGKG (SEQ ID NO:11), GSTSGSGKSSEGSGSTKG (SEQ ID NO:12), GSTSGSGKPGSGEGSTKG (SEQ ID NO:13), or EGKSSGSGSESKEF (SEQ ID NO:14). Linking moieties are described, for example, in Huston et al., Proc. Nat'l Acad. Sci 85:5879, 1988; Whitlow et al., Protein Engineering 6:989, 1993; and Newton et al., Biochemistry 35:545, 1996. Other suitable peptide linkers are those described in U.S. Pat. Nos. 4,751,180 and 4,935,233, which are hereby incorporated by reference.

The methods, compositions, and fusion polypeptides of the disclosure provide enhanced uptake and release of nucleic acid molecules by cells both *in vitro* and *in vivo.*

The term "therapeutic" is used in a generic sense and includes treating agents, prophylactic agents, and replacement agents. Examples of therapeutic molecules include, but are not limited to, cell cycle control agents; agents which inhibit cyclin proteins, such as antisense polynucleotides to the cyclin G1 and cyclin D1 genes; dsRNA that can be cleaved to provide siRNA molecules directed to specific growth factors such as, for example, epidermal growth factor (EGF), vascular endothelial growth factor (VEGF), erythropoietin, G-CSF, GM-CSF, TGF-α, TGF-β, and fibroblast growth factor; cytokines, including, but not limited to, Interleukins 1 through 13 and tumor necrosis factors; anticoagulants, anti-platelet agents; TNF receptor domains etc.

Using such methods and compositions, various diseases and disorders can be treated. For example, growth of tumor cells can be inhibited, suppressed, or destroyed upon delivery of an anti-tumor siRNA.

Thus, it is to be understood that the disclosure is not to be limited to any particular transduction domain or oligonucleotide/polynucleotide. Any anionically charged nucleic acid (*e.g*., dsRNA, siRNA and the like) can be delivered using the methods of the disclosure.

The polypeptides used in the disclosure (*e.g.*, with respect to particular domains of a fusion polypeptide or the full length fusion polypeptide) can comprise either the L-optical isomer or the D-optical isomer of amino acids or a combination of both. Polypeptides that can be used in the disclosure include modified sequences such as glycoproteins, retro-inverso polypeptides, D-amino acid modified polypeptides, and the like. A polypeptide includes naturally occurring proteins, as well as those which are recombinantly or synthetically synthesized. "Fragments" are a portion of a polypeptide. The term "fragment" refers to a portion of a polypeptide which exhibits at least one useful epitope or functional domain. The term "functional fragment" refers to fragments of a polypeptide that retain an activity of the polypeptide. For example, a functional fragment of a PTD includes a fragment which retains transduction activity. Biologically functional fragments, for example, can vary in size from a polypeptide fragment as small as an epitope capable of binding an antibody molecule, to a large polypeptide capable of participating in the characteristic induction or programming of phenotypic changes within a cell. An "epitope" is a region of a polypeptide capable of binding an immunoglobulin generated in response to contact with an antigen.

In some embodiments, retro-inverso peptides are used. "Retro-inverso" means an amino-carboxy inversion as well as enantiomeric change in one or more amino acids (*i.e.*, levantory (L) to dextrorotary (D)). A polypeptide of the disclosure encompasses, for example, amino-carboxy inversions of the amino acid sequence, amino-carboxy inversions containing one or more D-amino acids, and non-inverted sequence containing one or more D-amino acids. Retro-inverso peptidomimetics that are stable and retain bioactivity can be devised as described by Brugidou et al. (Biochem. Biophys. Res. Comm. 214(2): 685-693, 1995) and Chorev et al. (Trends Biotechnol. 13(10): 438-445, 1995).

In another aspect, the disclosure provides a method of producing a fusion polypeptide comprising a PTD domain, a nucleic acid binding domain (*e.g.,* DRBD) and a nucleic acid molecule by growing a host cell comprising a polynucleotide encoding the fusion polypeptide under conditions that allow expression of the polynucleotide, and recovering the fusion polypeptide. A polynucleotide encoding a fusion polypeptide of the disclosure can be operably linked to a promoter for expression in a prokaryotic or eukaryotic expression system. For example, such a polynucleotide can be incorporated in an expression vector to generate a fusion construct.

Accordingly, the disclosure also provides polynucleotides encoding a fusion protein construct of the disclosure. Such polynucleotides comprise sequences encoding one or more PTD domains, and/or a nucleic acid binding domain (e.g., DRBD). The polynucleotide may also encode linker domains that separate one or more of the PTDs and/or nucleic acid binding domains. In one aspect a fusion polypeptide comprising two or more PTD domains is produced and then linked to a charge reduced/protected oligonucleotide or polynucleotide.

Delivery of a polynucleotide of the disclosure can be achieved by introducing the polynucleotide into a cell using a variety of methods known to those of skill in the art. For example, a construct comprising such a polynucleotide can be delivered into a cell using a colloidal dispersion system. Alternatively, a polynucleotide construct can be incorporated (*i.e*., cloned) into an appropriate vector. For purposes of expression, the polynucleotide encoding a fusion polypeptide of the disclosure may be inserted into a recombinant expression vector. The term "recombinant expression vector" refers to a plasmid, virus, or other vehicle known in the art that has been manipulated by insertion or incorporation of a polynucleotide encoding a fusion polypeptide of the disclosure. The expression vector typically contains an origin of replication, a promoter, as well as specific genes that allow phenotypic selection of the transformed cells. Vectors suitable for such use include, but are not limited to, the T7-based expression vector for expression in bacteria (Rosenberg et al., Gene, 56:125, 1987), the pMSXND expression vector for expression in mammalian cells (Lee and Nathans, J. Biol. Chem., 263:3521, 1988), baculovirus-derived vectors for expression in insect cells, cauliflower mosaic virus, CaMV, and tobacco mosaic virus, TMV, for expression in plants.

Depending on the vector utilized, any of a number of suitable transcription and translation elements (regulatory sequences), including constitutive and inducible promoters, transcription enhancer elements, transcription terminators, and the like may be used in the expression vector (see, *e.g.,* Bitter et al., Methods in Enzymology, 153:516-544, 1987). These elements are well known to one of skill in the art.

The term "operably linked" and "operably associated" are used interchangeably herein to broadly refer to a chemical or physical coupling of two otherwise distinct domains that each have independent biological function. For example, operably linked refers to the functional linkage between a regulatory sequence and the polynucleotide regulated by the regulatory sequence. In another aspect, operably linked refers to the association of a nucleic acid domain and a transduction domain such that each domain retains its independent biological activity under appropriate conditions. Operably linked further refers to the link between encoded domains of the fusion polypeptides such that each domain is linked in-frame to give rise to the desired polypeptide sequence.

In yeast, a number of vectors containing constitutive or inducible promoters may be used (see, *e.g.,* Current Protocols in Molecular Biology, Vol. 2, Ed. Ausubel et al., Greene Publish. Assoc. & Wiley Interscience, Ch. 13, 1988; Grant et al., "Expression and Secretion Vectors for Yeast," in Methods in Enzymology, Eds. Wu & Grossman, Acad. Press, N.Y., Vol. 153, pp.516-544, 1987; Glover, DNA Cloning, Vol. II, IRL Press, Wash., D.C., Ch. 3, 1986; "Bitter, Heterologous Gene Expression in Yeast," Methods in Enzymology, Eds. Berger & Kimmel, Acad. Press, N.Y., Vol. 152, pp. 673-684, 1987; and The Molecular Biology of the Yeast Saccharomyces, Eds. Strathern et al., Cold Spring Harbor Press, Vols. I and II, 1982). A constitutive yeast promoter, such as ADH or LEU2, or an inducible promoter, such as GAL, may be used ("Cloning in Yeast," Ch. 3, R. Rothstein In: DNA Cloning Vol.11, A Practical Approach, Ed. DM Glover, IRL Press, Wash., D.C., 1986). Alternatively, vectors may be used which promote integration of foreign DNA sequences into the yeast chromosome.

An expression vector can be used to transform a host cell. By "transformation" is meant a permanent genetic change induced in a cell following incorporation of a polynucleotide exogenous to the cell. Where the cell is a mammalian cell, a permanent genetic change is generally achieved by introduction of the polynucleotide into the genome of the cell. By "transformed cell" or "recombinant host cell" is meant a cell into which (or into an ancestor of which) has been introduced, by means of molecular biology techniques, a polynucleotide encoding a fusion polypeptide of the disclosure. Transformation of a host cell may be carried out by conventional techniques as are known to those skilled in the art. where the host is prokaryotic, such as E. coli, competent cells which are capable of polynucleotide uptake can be prepared from cells harvested after exponential growth phase and subsequently treated by the CaCl2 method by procedures known in the art. Alternatively, MgCl2 or RbCl can be used. Transformation can also be performed after forming a protoplast of the host cell or by electroporation.

A fusion polypeptide of the disclosure can be produced by expression of polynucleotide encoding a fusion polypeptide in prokaryotes. These include, but are not limited to, microorganisms, such as bacteria transformed with recombinant bacteriophage DNA, plasmid DNA, or cosmid DNA expression vectors encoding a fusion polypeptide of the disclosure. The constructs can be expressed in E. coli in large scale. Purification from bacteria is simplified when the sequences include tags for one-step purification by nickel-chelate chromatography. Thus, a polynucleotide encoding a fusion polypeptide can also comprise a tag to simplify isolation of the fusion polypeptide. For example, a polyhistidine tag of, e.g., six histidine residues, can be incorporated at the amino terminal end of the fusion polypeptide. The polyhistidine tag allows convenient isolation of the protein in a single step by nickel-chelate chromatography. A fusion polypeptide of the disclosure can also be engineered to contain a cleavage site to aid in protein recovery the cleavage site may be part of a linker moiety as discussed above. A DNA sequence encoding a desired peptide linker can be inserted between, and in the same reading frame as, a polynucleotide encoding a PTD, or fragment thereof followed by a nucleic acid binding domain, the PTD may also be linked to a desired nucleic acid (*e.g*., dsRNA, DNA, siRNA, and the like), using any suitable conventional technique. For example, a chemically synthesized oligonucleotide encoding the linker can be ligated between two coding polynucleotides. In particular embodiments, a polynucleotide of the disclosure will encode a fusion polypeptide comprising from two to four separate domains (*e.g*., one or more PTD domain and one or more a nucleic acid domains) separated by linkers. In some embodiments, once purified, a fusion polypeptide comprising a plurality of PTDs is associated or linked with an oligonucleotide comprising a phosphodiester and/or phosphothioate protecting group or other anionic charge reducing group.

When the host cell is a eukaryotic cell, such methods of transfection of DNA as calcium phosphate co-precipitates, conventional mechanical procedures, such as microinjection, electroporation, insertion of a plasmid encased in liposomes, or virus vectors may be used. Eukaryotic cells can also be cotransfected with a polynucleotide encoding the PTD-fusion polypeptide of the disclosure, and a second polynucleotide molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the fusion polypeptide (see, *e.g.*, Eukaryotic Viral Vectors, Cold Spring Harbor Laboratory, Gluzman ed., 1982).

Eukaryotic systems, and typically mammalian expression systems, allow for proper post-translational modifications of expressed mammalian proteins to occur. Eukaryotic cells that possess the cellular machinery for proper processing of the primary transcript, glycosylation, phosphorylation, and advantageously secretion of the fusion product can be used as host cells for the expression of the PTD-fusion polypeptide of the disclosure. Such host cell lines may include, but are not limited to, CHO, VERO, BHK, HeLa, COS, MDCK, Jurkat, HEK-293, and WI38.

For long-term, high-yield production of recombinant proteins, stable expression is used. Rather than using expression vectors that contain viral origins of replication, host cells can be transformed with the cDNA encoding a fusion polypeptide of the disclosure controlled by appropriate expression control elements (*e.g*., promoter, enhancer, sequences, transcription terminators, polyadenylation sites, and the like), and a selectable marker. The selectable marker in the recombinant plasmid confers selectivity (*e.g*., by cytotoxin resistance) and allows cells to stably integrate the plasmid into their chromosomes and grow to form foci that, in turn, can be cloned and expanded into cell lines. For example, following the introduction of foreign DNA, engineered cells may be allowed to grow for 1-2 days in an enriched media, and then are switched to a selective media. A number of selection systems may be used, including, but not limited to, the herpes simplex virus thymidine kinase (Wigler et al., Cell, 11:223, 1977), hypoxanthine-guanine phosphoribosyltransferase (Szybalska & Szybalski, Proc. Natl. Acad. Sci. USA, 48:2026, 1962), and adenine phosphoribosyltransferase (Lowy et al., Cell, 22:817, 1980) genes can be employed in tk-, hgprt- or aprt- cells, respectively. Also, antimetabolite resistance can be used as the basis of selection for dhfr, which confers resistance to methotrexate (Wigler et al., Proc. Natl. Acad. Sci. USA, 77:3567, 1980; O'Hare et al., Proc. Natl. Acad. Sci. USA, 8:1527, 1981); gpt, which confers resistance to mycophenolic acid (Mulligan & Berg, Proc. Natl. Acad. Sci. USA, 78:2072, 1981; neo, which confers resistance to the aminoglycoside G-418 (Colberre-Garapin et al., J. Mol. Biol., 150:1, 1981); and hygro, which confers resistance to hygromycin genes (Santerre et al., Gene, 30:147, 1984). Additional selectable genes have been described, namely trpB, which allows cells to utilize indole in place of tryptophan; hisD, which allows cells to utilize histinol in place of histidine (Hartman & Mulligan, Proc. Natl. Acad. Sci. USA, 85:8047, 1988); and ODC (ornithine decarboxylase), which confers resistance to the ornithine decarboxylase inhibitor, 2-(difluoromethyl)-DL-ornithine, DFMO (McConlogue L., In: Current Communications in Molecular Biology, Cold Spring Harbor Laboratory, ed., 1987).

Techniques for the isolation and purification of either microbially or eukaryotically expressed PTD-fusion polypeptides of the disclosure may be by any conventional means, such as, for example, preparative chromatographic separations and immunological separations, such as those involving the use of monoclonal or polyclonal antibodies or antigen.

The fusion polypeptides of the disclosure are useful for the delivery of anionically charged nucleic acid molecules (*e.g.,* dsRNA, siRNA, DNA, antisense, ribozymes and the like) for the treatment and/or diagnosis of a number of diseases and disorders. For example, the fusion polypeptides can be used in the treatment of cell proliferative disorders, wherein the protected oligo- or polynucleotide is reversibly modified such that it traverses a cell membrane alone or in associate with a PTD to target genes that induce cell proliferation. The PTD domain increases the overall net cationic charge or reduces the overall net anionic charge of the nucleic acid construct facilitating facilitates uptake by the cell. Thus, the constructs are useful for treatment of cells having cell proliferative disorders. Similarly, the constructs of the disclosure can be used to treat inflammatory diseases and disorders, infections, vascular disease and disorders and the like.

In one aspect, the construct of the disclosure may alternatively comprise, or in addition to, the PTD, a targeting domain. The targeting domain can be a receptor, receptor ligand or antibody useful for directing the construct to a particular cell type that expresses the cognate binding domain.

Typically a construct of the disclosure will be formulated with a pharmaceutically acceptable carrier, although the fusion polypeptide may be administered alone, as a pharmaceutical composition.

A pharmaceutical composition according to the disclosure can be prepared to include a fusion polypeptide of the disclosure, into a form suitable for administration to a subject using carriers, excipients, and additives or auxiliaries. Frequently used carriers or auxiliaries include magnesium carbonate, titanium dioxide, lactose, mannitol and other sugars, talc, milk protein, gelatin, starch, vitamins, cellulose and its derivatives, animal and vegetable oils, polyethylene glycols and solvents, such as sterile water, alcohols, glycerol, and polyhydric alcohols. Intravenous vehicles include fluid and nutrient replenishers. Preservatives include antimicrobial, anti-oxidants, chelating agents, and inert gases. Other pharmaceutically acceptable carriers include aqueous solutions, non-toxic excipients, including salts, preservatives, buffers and the like, as described, for instance, in Remington's Pharmaceutical Sciences, 15th ed., Easton: Mack Publishing Co., 1405-1412, 1461-1487 (1975), and The National Formulary XIV., 14th ed., Washington: American Pharmaceutical Association (1975), the contents of which are hereby incorporated by reference. The pH and exact concentration of the various components of the pharmaceutical composition are adjusted according to routine skills in the art. See Goodman and Gilman's, The Pharmacological Basis for Therapeutics (7th ed.).

The pharmaceutical compositions according to the disclosure may be administered locally or systemically. By "therapeutically effective dose" is meant the quantity of a fusion polypeptide according to the disclosure necessary to prevent, to cure, or at least partially arrest the symptoms of a disease or disorder (*e.g.,* to inhibit cellular proliferation). Amounts effective for this use will, of course, depend on the severity of the disease and the weight and general state of the subject. Typically, dosages used *in vitro* may provide useful guidance in the amounts useful for in situ administration of the pharmaceutical composition, and animal models may be used to determine effective dosages for treatment of particular disorders. Various considerations are described, *e.g.,* in Langer, Science, 249: 1527, (1990); Gilman *et al.* (eds.) (1990), each of which is herein incorporated by reference.

As used herein, "administering a therapeutically effective amount" is intended to include methods of giving or applying a pharmaceutical composition of the disclosure to a subject that allow the composition to perform its intended therapeutic function. The therapeutically effective amounts will vary according to factors, such as the degree of infection in a subject, the age, sex, and weight of the individual. Dosage regima can be adjusted to provide the optimum therapeutic response. For example, several divided doses can be administered daily or the dose can be proportionally reduced as indicated by the exigencies of the therapeutic situation.

The pharmaceutical composition can be administered in a convenient manner, such as by injection (*e.g*., subcutaneous, intravenous, and the like), oral administration, inhalation, transdermal application, or rectal administration. Depending on the route of administration, the pharmaceutical composition can be coated with a material to protect the pharmaceutical composition from the action of enzymes, acids, and other natural conditions that may inactivate the pharmaceutical composition. The pharmaceutical composition can also be administered parenterally or intraperitoneally. Dispersions can also be prepared in glycerol, liquid polyethylene glycols, and mixtures thereof, and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions or dispersions. The composition will typically be sterile and fluid to the extent that easy syringability exists. Typically the composition will be stable under the conditions of manufacture and storage and preserved against the contaminating action of microorganisms, such as bacteria and fungi. The carrier can be a solvent or dispersion medium containing, for example, water, ethanol, polyol (for example, glycerol, propylene glycol, and liquid polyetheylene glycol, and the like), suitable mixtures thereof, and vegetable oils. The proper fluidity can be maintained, for example, by the use of a coating, such as lecithin, by the maintenance of the required particle size, in the case of dispersion, and by the use of surfactants. Prevention of the action of microorganisms can be achieved by various antibacterial and antifungal agents, for example, parabens, chlorobutanol, phenol, ascorbic acid, thimerosal, and the like. In many cases, isotonic agents, for example, sugars, polyalcohols, such as mannitol, sorbitol, or sodium chloride are used in the composition. Prolonged absorption of the injectable compositions can be brought about by including in the composition an agent that delays absorption, for example, aluminum monostearate and gelatin.

Sterile injectable solutions can be prepared by incorporating the pharmaceutical composition in the required amount in an appropriate solvent with one or a combination of ingredients enumerated above, as required, followed by filtered sterilization. Generally, dispersions are prepared by incorporating the pharmaceutical composition into a sterile vehicle that contains a basic dispersion medium and the required other ingredients from those enumerated above.

The pharmaceutical composition can be orally administered, for example, with an inert diluent or an assimilable edible carrier. The pharmaceutical composition and other ingredients can also be enclosed in a hard or soft-shell gelatin capsule, compressed into tablets, or incorporated directly into the subject's diet. For oral therapeutic administration, the pharmaceutical composition can be incorporated with excipients and used in the form of ingestible tablets, buccal tablets, troches, capsules, elixirs, suspensions, syrups, wafers, and the like. Such compositions and preparations should contain at least 1% by weight of active compound. The percentage of the compositions and preparations can, of course, be varied and can conveniently be between about 5% to about 80% of the weight of the unit.

The tablets, troches, pills, capsules, and the like can also contain the following: a binder, such as gum gragacanth, acacia, corn starch, or gelatin; excipients such as dicalcium phosphate; a disintegrating agent, such as corn starch, potato starch, alginic acid, and the like; a lubricant, such as magnesium stearate; and a sweetening agent, such as sucrose, lactose or saccharin, or a flavoring agent such as peppermint, oil of wintergreen, or cherry flavoring. When the dosage unit form is a capsule, it can contain, in addition to materials of the above type, a liquid carrier. Various other materials can be present as coatings or to otherwise modify the physical form of the dosage unit. For instance, tablets, pills, or capsules can be coated with shellac, sugar, or both. A syrup or elixir can contain the agent, sucrose as a sweetening agent, methyl and propylparabens as preservatives, a dye, and flavoring, such as cherry or orange flavor. Of course, any material used in preparing any dosage unit form should be pharmaceutically pure and substantially non-toxic in the amounts employed. In addition, the pharmaceutical composition can be incorporated into sustained-release preparations and formulations.

Thus, a "pharmaceutically acceptable carrier" is intended to include solvents, dispersion media, coatings, antibacterial and antifungal agents, isotonic and absorption delaying agents, and the like. The use of such media and agents for pharmaceutically active substances is well known in the art. Except insofar as any conventional media or agent is incompatible with the pharmaceutical composition, use thereof in the therapeutic compositions and methods of treatment is contemplated. Supplementary active compounds can also be incorporated into the compositions.

It is especially advantageous to formulate parenteral compositions in dosage unit form for ease of administration and uniformity of dosage. "Dosage unit form" as used herein, refers to physically discrete units suited as unitary dosages for the subject to be treated; each unit containing a predetermined quantity of pharmaceutical composition is calculated to produce the desired therapeutic effect in association with the required pharmaceutical carrier. The specification for the dosage unit forms of the disclosure are related to the characteristics of the pharmaceutical composition and the particular therapeutic effect to be achieve.

The principal pharmaceutical composition is compounded for convenient and effective administration in effective amounts with a suitable pharmaceutically acceptable carrier in an acceptable dosage unit. In the case of compositions containing supplementary active ingredients, the dosages are determined by reference to the usual dose and manner of administration of the said ingredients.

### EXAMPLES

### RNB Synthesis example.

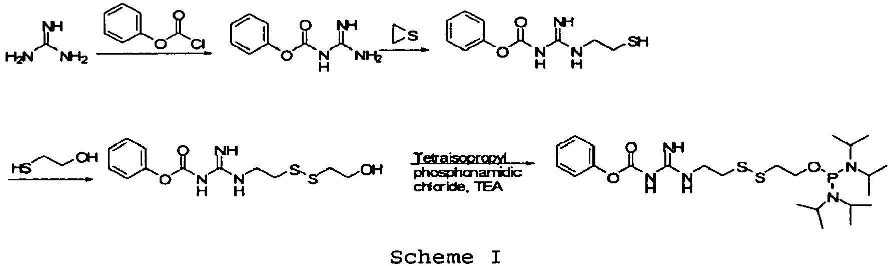

Phenyl chloroformate is added to Guanidine hydrochloride under basic conditions. Selective ring opening of thirane is accomplished under lewis acidic conditions to provide the free thiol. Thiolated material is linked to betamercaptoethanol via a disulfide linkage to provide a free alcohol. Reaction of the free alcohol with tetraisopropylphosphonamidic chloride provides the diamidite capable of reaction with the free 3'OH of any nucleoside amidite precursor. Oligonucleotides were synthesized using techniques known in the art as described herein.

### RNN Phosphoramidite protection.

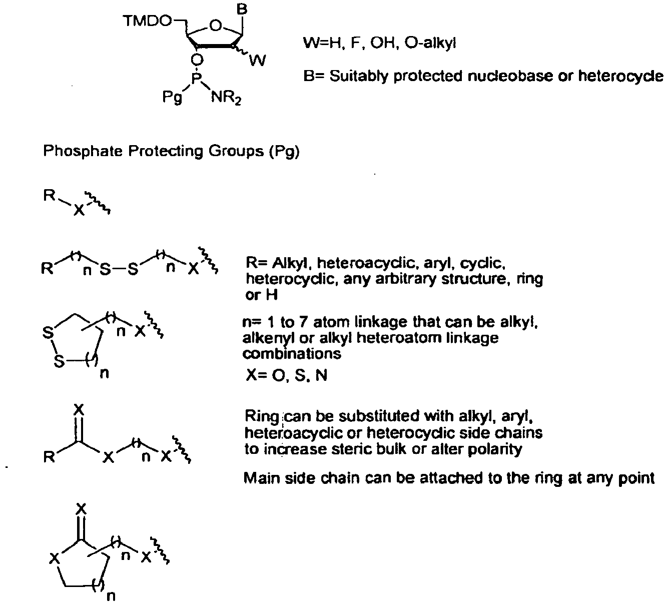

### Ribonucleic Base RNB protecting groups.

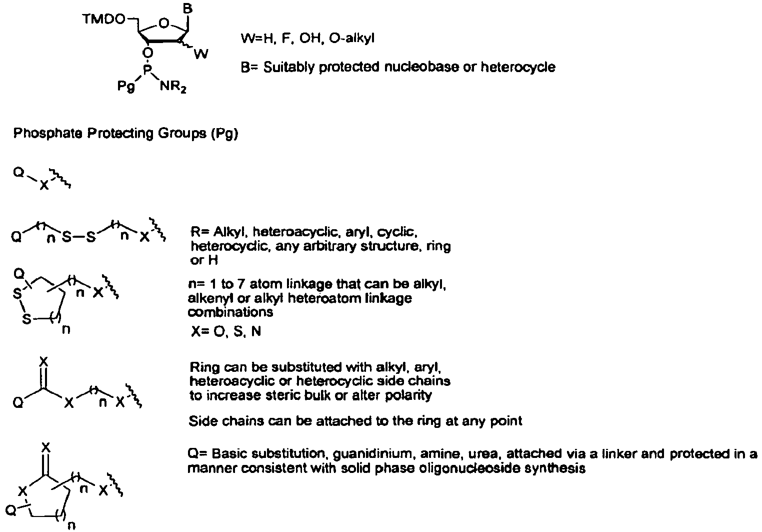

Reactions were carried out under an argon atmosphere. Glassware was cleaned overnight in a KOH/EtOH base bath, rinsed with MeOH and flame dried under vacuum before use in all anhydrous systems. Reactions were run with solvents that were either purchased sure-sealed over molecular sieves or were distilled using protocols listed in Purification of Laboratory Chemicals 4th ed. and stored over sieves.

Tetrahydrofuran (THF) was distilled from sodium metal and benzophenone, triethyl amine (Et₃N), di-isopropyl amine (DIEA) and pyridine (py) were distilled from sodium metal. Dichloromethane (CH₂Cl₂), methanol (MeOH) and toluene were distilled from calcium hydride. All other solvents and reagents were purchased from Fisher Chemical Co., Aldrich Chemical Co., EMD or Acros Organics and used without further purification. Reactions were cooled to -78°C *via* dry ice-acetone baths.

Flash column chromatography was performed using Merck grade 60 silica gel (230-400 mesh) and TLC analysis was carried out using Merck 60F-254 pre-coated silica sheets. Visualization of TLC plates was achieved using ultraviolet light, *p*-anisaldehyde in ethanol with sulfuric acid, polyphosphomolybdic acid and cerium sulfate in EtOH with H₂SO₄, ninhydrin in EtOH with H₂SO₄, potassium permanganate or iodine. Solvent removal was effected by Buchi rotary evaporator equipped with a dry ice isopropanol cold finger trap, and a H₂O aspirator was used to concentrate *in vacuo.* Samples were further dried under reduced pressure on a high vacuum line over P₂O₅ and KOH. ¹H NMR spectra were taken on a Varian Unity Inova 400 in CDCl₃ or *d*₆Benzene at ambient temperature unless otherwise noted. Mestre-C was used to visualize and measure J couplings. ¹H chemical shifts in CDCl₃ were reported in ppm; (δ units) downfield from tetramethylsilane. ¹H NMR splitting patterns are designated as a singlet (s), doublet (d), triplet (t) or quartet (q). All first order splitting patterns were assigned based on the appearance of the multiplet as interpreted by the program. Splitting patterns that could not be easily interpreted are designated as multiplet (m) or (br). In cases where broad or multiplet splitting patterns are clearly centered on one peak the chemical shift for that peak was reported instead of a range. The solvent peak at 2.49 was used as an internal reference in DMSO-d₆, and the solvent peak at 3.30 was used as an internal reference in CD₃OD. Solvent peaks were used as internal references for all ¹³C NMR. NMR spectral data is tabulated as follows: chemical shift, multiplicity, coupling constant and number of protons. ³¹P NMR chemical shifts were measured relative to a phosphoric acid standard and spectra were acquired in *d*₆ benzene unless otherwise noted. NMR ¹³C and ¹H data were not reported when the product was a racemic mixture. Mass spectroscopy was obtained at the HHMI Analytical Instrumentation Center (AIC) of the University of California San Diego.

Oligonucleotides of 17-21 nt in length were synthesized on an MerMade 6 automated DNA/RNA synthesizer. Glen Research Q CPG support was used with ethylthioltetrazole as the coupling reagent during 3 coupling steps of 5 minutes each. Phosphine was oxidized to phosphate by the standard iodine method and capping was performed with phenoxyacetic anhydride. All amidites and materials used on the MerMade 6 synthesizer were either synthesized or purchased from EMD, ChemGenes or Glen Research.

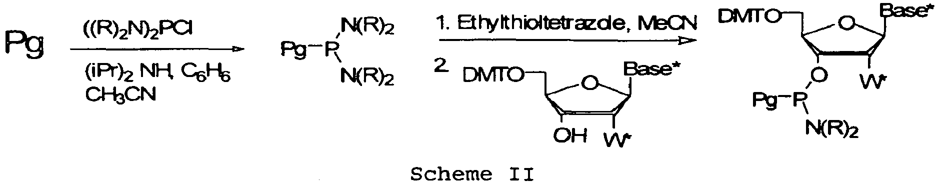

Examples of alcohol synthesis (Scheme II). Modified amidites were synthesized by preparing the free alcohol or thiol in the configurations shown below.

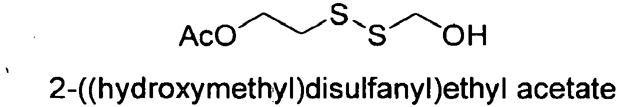

**VI_019:** 2 hydroxyethyl disulfide (1.2ml, 10mmol), Imidazole (1.23g, 10mmol) and DMF were placed in a flask equipped with a stir bar at room temperature. Acetylchloride (639.4g, 9mmol) was dissolved in DMF and added over 2h by syringe pump to the stirring solution. After 6 h the solution was dissolved in EtOAc and extracted with saturated sodium bicarbonate, brine and dried over sodium sulfate. Ethyl acetate was removed and the resulting oil was fractionated over a silica column. Fractions containing product were pooled and solvent was removed to give 635mg of a colorless oil at 36%.

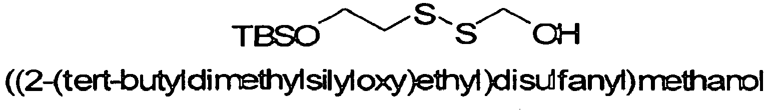

**VI_139:** 2 hydroxyethyl disulfide (19.17g. 160mmol), Imidazole (20.7g, 304mmol) and DMF (100ml) were placed in a flask equipped with a stir bar at room temperature. TBSC1 (38.9ml, 152mmol) was dissolved in DMF (60ml) and added over 2h by syringe pump to the stirring solution. After 15 h the solution was dissolved in EtOAc and extracted with sodium bicarbonate, brine and dried over sodium sulfate. Evaporation gave an oil that was applied to a silica column. Fractions containing product were pooled and evaporated to give 18.8g of a light amber oil at 31%. (M+H)=268.74. 1H NMR (500 MHz, *Solvent*) δ ppm 3.92-3.80 (m, 4H), 2.90-2.75 (m, 4H), 2.50 (s, 1H), 0.92-0.82 (m, 9H), 0.05 (m, 6H).

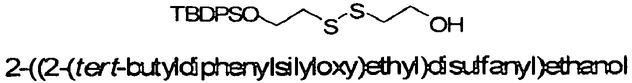

**VI_063:** 2 hydroxyethyl disulfide (2.4ml, 20mmol), Imidazole 5.8g, 85.5mmol) and 20ml DMF were placed in a flask equipped with a stir bar at room temperature. TBDPSCl (4.9ml, 19mmol) was dissolved in DMF and added over 2h by syringe pump to the stirring solution. After 15 h the solution was dissolved in EtOAc and extracted. Silica chromatography yielded 1.79g of a light amber oil at 24%. ¹H NMR (500 MHz, *CDCl₃*) δ ppm 7.82-7.64 (m, 1H), 7.50-7.38 (m, 1H), 5.32-5.30 (m, 1H), 3.99-3.91 (m, 1H), 3.91-3.84 (m, 1H), 2.93-2.87 (m, 1H), 2.81-2.77 (m, 1H), 2.34-2.31 (m, 1H), 1.13-1.08 (m, 1H).

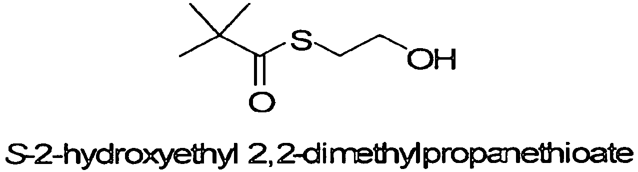

**VI_183:** Mercaptoethanol (10ml, 142.6mmol) and Triethyl amine (19.9ml, 142.6mmol) were dissolved in dichloromethane 275ml and cooled to -78°C. Pivaloyl chloride (17.56ml, 142.6mmol) was added dropwise to the stirring solution. The reaction was allowed to proceed for one hour at -78°C then it was warmed to rt and stirred for an additional 1h before quenching with 150ml water. The aqueous was washed (3x100 ml DCM) and dried with magnesium sulfate. Quantitative conversion was observed by TLC crude was diluted with Hexanes and run over a short silica column to give 19.6g of colorless oil at 85%. ¹H NMR (400 MHz, *CDCl₃)* δppm 3.61 (dd, *J* = 11.79 Hz, 2H), 3.05 (t, *J* = 5.46 Hz, 1H), 2.95 (td, *J* = 12.60, 3.42 Hz, 2H), 1.14 (d, *J* = 6.18 Hz, 9H). ¹³C NMR (101 MHz, *CDCl₃* δ ppm (207.2, 61.4, 46.3, 31.0, 27.1 ppm.

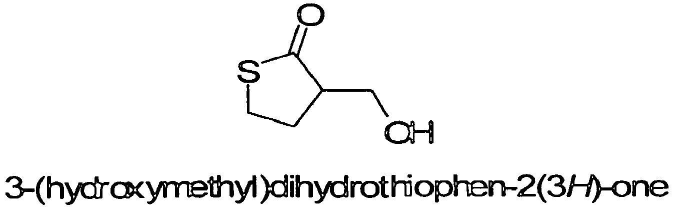

**VI_269:** Thiobutyrolactone (5.1g, 50mmol) in dry THF was added dropwise to a stirred solution of lithium diisopropylamide. (Diisopropylamine (5.1mg, 50.5mmol) and n-butyllithium in hexane(31.3ml, 50mmol) at -78C). The resulting solution was stirred for 10 minutes at which time formaldehyde (31.3g) carried in a stream of argon was added (Formaldehyde was formed by heating p-formaldehyde to 150C) The reaction was allowed to proceed for 2.5h at -78C. The formaldehyde stream was removed and reaction was allowed to proceed for an additional 30m. The reaction was quenched by the addition of (-100ml) 1M HCl at -78C then allowed to warm to rt and filtered through a bed of celite. The filtrate was extracted with ethyl acetate 5X100ml and the combined organic layers were dried (Na₂SO₄) and concentrated to an oil. The oil was chromatographically isolated to give 1.1g of isolate at 17%.

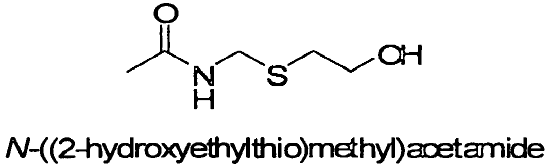

**IV_185:** Acetamide (890mg, 10mmol) was dissolved in TFA (10ml) at rt. Mercaptoethanol (700mg, 10mmol) was added dropwise to the stirring solution. The reaction was allowed to proceed for 30m. TFA was evaporated and the resulting oil was recrystallized from aqueous 2 propanol.

### Examples of Diamidite formation:

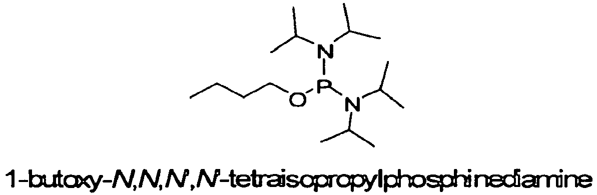

**VI_173:** n-butanol (190ul, 1.0mmol), triethylamine (418ul, 3mmol), and benzene were placed in a flask equipped with a stir bar at room temperature. Chlorophosphine (292mg, 1.1mmol) was added to the stirring solution and the reaction was allowed to progress for 2h. Solvent was removed from the resulting mixture and the oil was applied directly to a triethylamine pretreated column. Fractions containing product were evaporated to give 311mg oil at 64%.

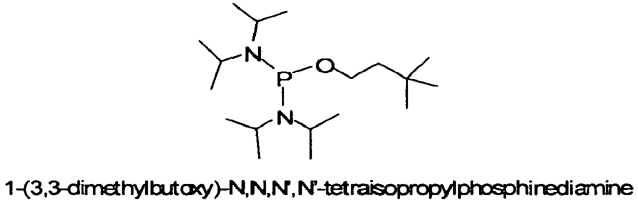

**VI_257:** Tetraisopropyl phosphoramidic chloride (2.0g, 7.5mmol) was transferred under a stream of argon to a flame dried reaction vessel equipped with a stir bar at room temperature. Triethylamine (1.05ml, 7.5mmol) and benzene (7ml) were added via syringe. The reaction was started by addition of 2'2' dimethyl butanol (700mg, 6.84mmol) by syringe. The reaction was monitored for complete conversion by TLC over the course of 2.5h. Solvent was removed from the mixture and the resulting oil was applied directly to a TEA pretreated column. Solvent was removed from fractions containing product and the resulting oil was co-evaporated 2X with toluene and lyophilized from benzene overnight to give 1.6g colorless solid at 71%.

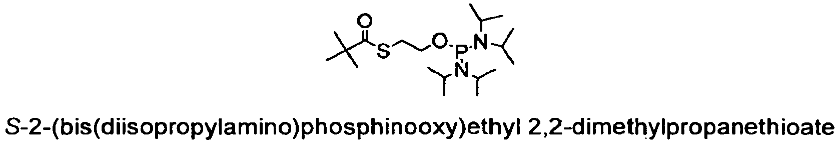

**VI_253 SPTE:** Tetraisopropyl phosphoramidic chloride(5.0g, 18.81mmol) was transferred under a stream of argon to a flame dried reaction vessel equipped with a stir bar at room temperature. Triethylamine (2.6ml, 18.8mmol) and benzene (20ml) were added via syringe. The reaction was started by addition of the thioester VI_183 (2.77g, 17.1mmol) by syringe. The reaction was monitored for complete conversion by TLC over the course of 2.5h. Solvent was removed from the mixture and the resulting oil was applied directly to a TEA pretreated column. Solvent was removed from fractions containing product and the resulting oil was co-evaporated 2X with toluene and lyophilized from benzene overnight to give 6.08g colorless solid at 91%. ³¹P NMR (162 MHz, *d₆Benzene* δ ppm 123.5 ppm. ¹³C NMR (101 MHz, *CDCl₃* δ ppm 205.2, 63.6, 63.4, 44.7, 44.6, 30.8, 30.7, 27.4, 24.7, 24.6, 24.0, 24.0 ppm. 1H NMR (400 MHz, *Solvent* δ ppm 3.70 (dd, *J* = 13.46 Hz, 2H), 3.48 (dq, *J* = 13.44 Hz, 4H), 3.14 (t, *J* = 6.34 Hz, 2H), 1.25-1.11 (m, 33H).

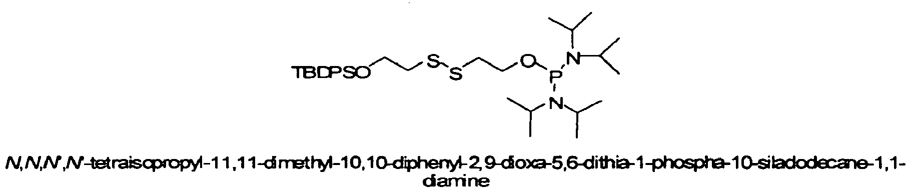

**VI_167:** Bis (diisopropylamino) chlorophosphine (2g, 7.5mmol) IV_139 (3.02g, 7.7mmol) and diisopropyl amine (2ml, 15mmol) were reacted in 10ml benzene. After chromatography 4.0g of oil was collected at 86%.

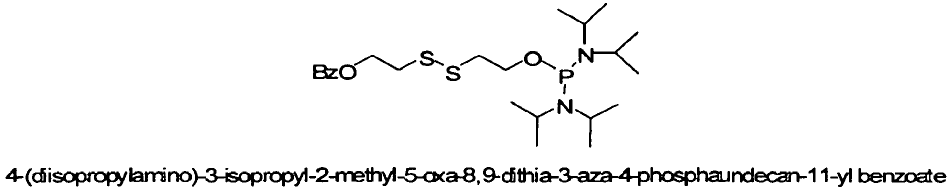

**VI_171:** 2 hydroxyethyl disulfide (910ml, 7.6mmol), Triethylamine (3.2ml, 22.8mmol), and benzene were placed in a flask equipped with a stir bar at room temperature. To the stirring solution was added benzoyl chloride (970ul, 8.36mmol). The reaction was monitored for complete conversion of disulfide by TLC. The reaction was diluted with 10ml ether and filtered into a fresh reaction vessel under anhydrous conditions. Chlorophosphine (lg, 3.76mmol) was added dry under a stream of argon to the stirring solution and the reaction allowed to progress for an additional 2h. Solvent was removed and the resulting oil was applied directly to a TEA pretreated column. Solvent was removed from collected fractions to give 648mg of a colorless oil at 35%.

### Error! Objects cannot be created from editing field codes.

**VI_147:** Nucleoside(132.2mg, 0.2mmol) and diisopropylethylamine amine (105ul, 0.6mmol) were diluted with DCM. This solution was slowly added to a dilution of phosphorus trichloride 2M in dichloromethane (100ul) dropwise. Flask was equipped with a vent needle. Completion conversion of starting material was observed at by TLC. Solvent and excess phosphorus trichloride were removed from the reaction mixture by evaporation. The reaction was resuspended in anhydrous ether and filtered to remove amine salts and unreacted reacted phosphine. The ether was evaporated and the resulting crude solid was taken on to the next step of the reaction series directly due to issues of spontaneous decomposition.

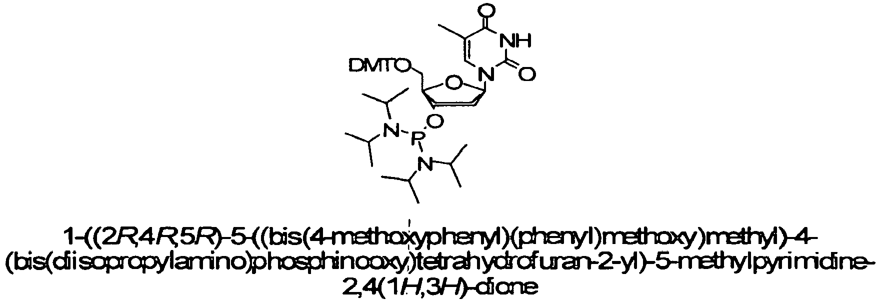

**VI_179:** Nucleoside (1.53g, 2.81mmol) and diisopropylethylamine amine (1.0ml, 5.63mmol) were diluted with 10ml dichloromethane. This solution was slowly added to a dilution of chloroamidite (997mg, 3.75mmol) in 10ml dichloromethane in a dropwise manner. The reaction was allowed to proceed for 30 min at which point a vent needle was placed in the flask to allow HCl to escape with the argon flow. The reaction vessel was heated to with a heat gun and the vent was removed. Completion conversion of starting material was observed at by TLC. Solvent was removed from the reaction mixture the reaction was resuspended in anhydrous ether and filtered to remove amine salts and unreacted chloroamidite. At 4h the reaction was quenched with 1ml TEA and evaporated to give an oil that was loaded directly onto a 1%TEA pretreated silica plug. Fractions containing product were pooled and evaporated to give 2.86g of a pale yellow solid at 85%. ³¹P NMR (162 MHz, *d₆Benzene*) δ ppm 115.1.

Example of nucleoside activation as an phosphotriester amidite for solid phase synthesis: Nucleoside amidites were prepared in a manner suitable for the Caruthers method of solid phase oligonucleotide synthesis. Nucleoside and activator are added to a flame dried vial. Phosphoramidite is added to the stirring solution reaction was allowed to proceed for a minimum of 3h. Solvent was removed from the reactions and the resulting foam or oil was applied directly to a triethylamine pretreated silica column in running buffer. Fractions containing only product were pooled and the solvent was removed. The resulting foam was dissolved in acetonitrile and syringe filtered (0.45um). Acetonitrile was removed and the resulting foam was dissolved in benzene and lyophilized to remove all traces of triethyl amine and residual water. Samples were sealed in glass containers under argon and stored at -20°C prior to use on the automated oligonucleoside synthesizer.

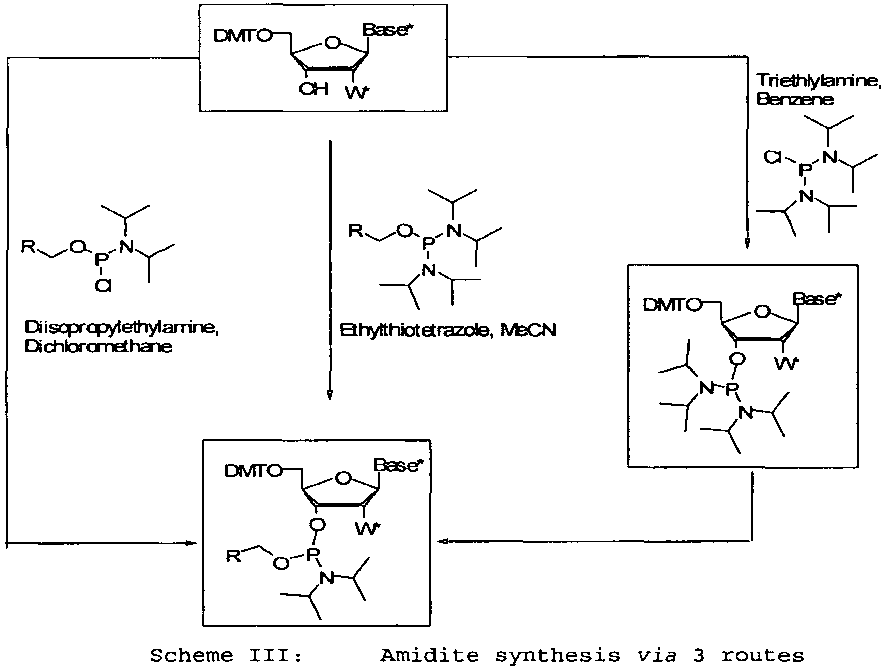

In some aspect, phenooxyacetal base protection was used during synthesis. In others, a hindered base for cleavage form the solid support, which did not use a photolabile linker was used. One advantage of the disclosure is the ability to use either or both of DNA and RNA nucleotides.

### Synthetic route with 2'F and 2'2' dimethyl butanol phosphate protection:

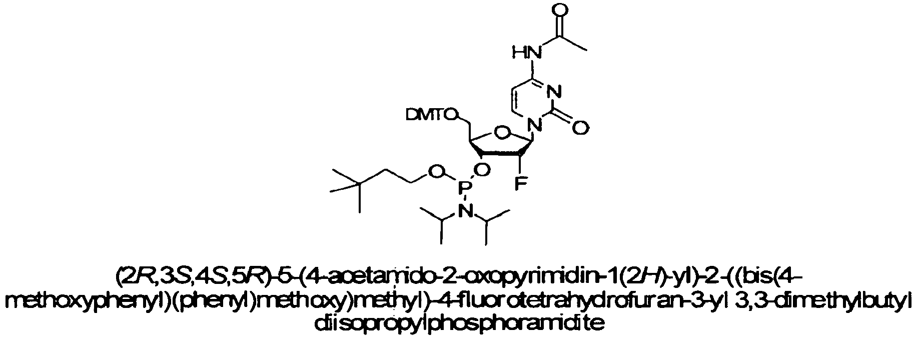

**VI_261:** Protected cytosine (840mg, 1.43mmol) and 2'2'dimethyl phosphordiamidite VI_257 (498.5mg, 1.5mmol) were dissolved in 5.7 ml dichloromethane. Ethylthiotetrazole 0.45M in acetonitrile (5.7ml) was added and the reaction was found to be complete in 2.5h. Chromatography and filtration resulted in 800mg lyophilate at 68.4%.

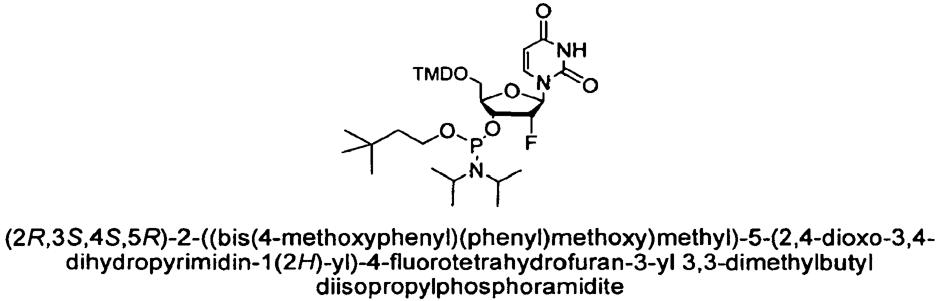

**VI_261:** Protected uridine (782mg, 1.43mmol) and 2'2'dimethyl phosphordiamidite VI_257 (498.5mg, 1.5mmol) were dissolved in 5.7 ml dichloromethane. Ethylthiotetrazole 0.45M in acetonitrile (5.7ml) was added and the reaction was found to be complete in 2.5h. Chromatography and filtration resulted in 860mg lyophilate at 77.4%.

### Synthetic route to 2'F methylphosphoramidites:

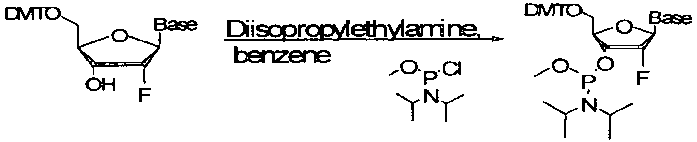

Commercially available, (Carbosynth, R.I. Chemical) free 3'OH nucleobases were reacted directly with the chlorophosphine under basic conditions in benzene.

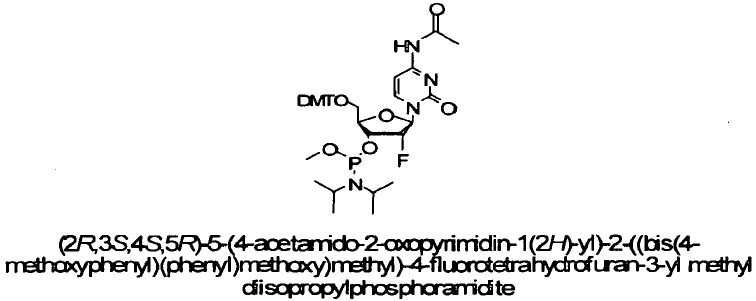

**VI_245, 237:** Protected cytosine nucleoside (1.0g, 1.7mmol) and DIEA (1.42ml, 8.2mmol) were added to a flame dried vial. To the stirring solution was added phosphonamidic chloride (1.34g, 6.79mmol) and the reaction was allowed to proceed for 3h. The reaction was concentrated to an oil and applied directly onto a TEA pretreated column. Isolation was achieved by chromatography DCM:Ethyl acetate:TEA 45:45:10. Fractions containing product were evaporated followed by transfer/filtration through a 0.45um filter in acetonitrile. Lyophilization from benzene gave 1.02g lyophilate at 80.4%.

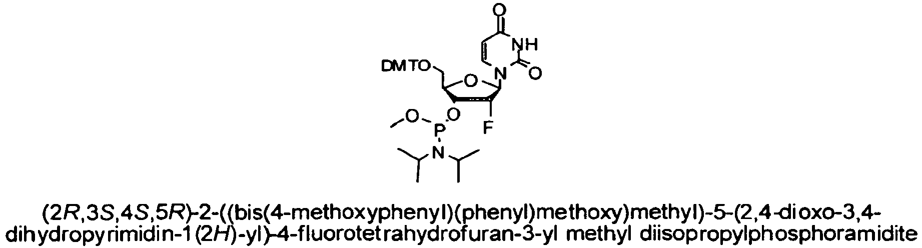

**VI_251:** Protected Uridine nucleoside (2.74g, 5.0 mmol) and DIEA (4.2ml, 24 mmol) were added to a flame dried vial. To the stirring solution was added phosphonamidic chloride (3.94g, 20.0 mmol) and the reaction was allowed to proceed for 3h. The reaction was concentrated to an oil and applied directly onto a TEA pretreated column. Isolation was achieved by chromatography Hexane: DCM:Ethyl acetate:TEA 45:45:45:10. Fractions containing product were evaporated followed by transfer/filtration through a 0.45um filter in acetonitrile. Lyophilization from benzene gave 2.46g lyophilate at 84%.

### Synthetic Route with SPTE biolabile protecting groups:

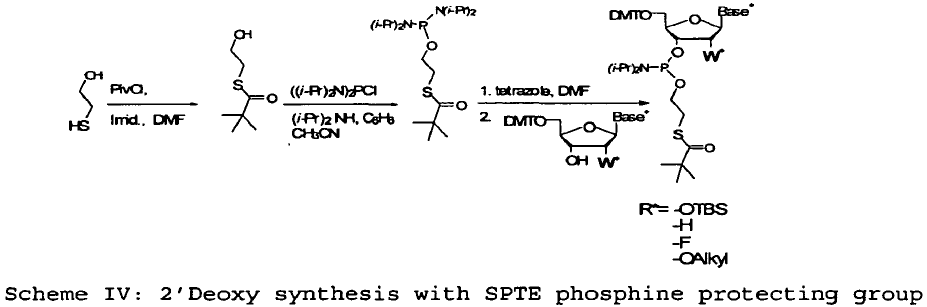

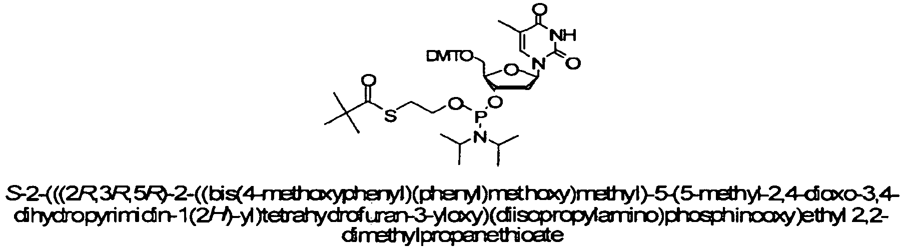

**VI_247:** Protected Nucleoside (2.07g, 3.08mmol) and ethylthiotetrazole (15.2 ml) were diluted with DCM. To the stirring solution was added the phosphorodiamidite VI_253 (1.57g, 4.0mmol) and the reaction was allowed to proceed for 30m. The reaction was quenched by pouring the crude into a saturated solution of sodium bicarbonate and washing with brine washed 3X10ml. The organic was dried over NaSO4 and evaporated.
Isolation was achieved by chromatography
Hexane:DCM:EthylAcetate:TEA 40:40:20:7.5. Fractions containing product were evaporated followed by transfer/filtration through a 0.45um filter in benzene and lyophilization from benzene overnight to ensure complete removal of water. 2.74g lyophilate was recovered at 86%. ³¹P NMR (162 MHz, *d₆Benzene*) δ ppm 148.7, 148.2.

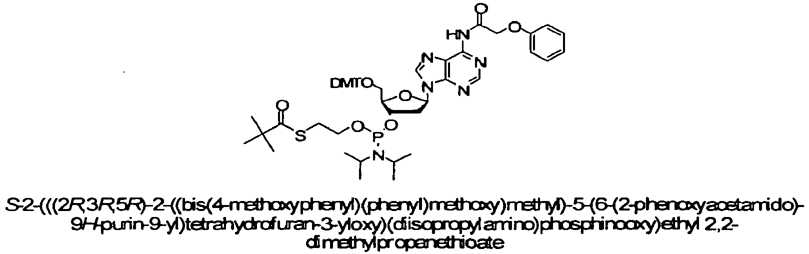

**VI_267:** 5' DMT phenoxyacyldeoxyadenosine (3.92g, 5.7mmol) and ethylthiotetrazole activator (22.8ml, 5.7mmoles) were added to a flame dried vial and diluted in dichlomethane (22.8ml). To the stirring solution was added the phosphorodiamidite VI_253 (2.36g, 6.0mmol) and the reaction was allowed to proceed for 3h. Solvent was removed from the reaction vessel and loaded directly on a TEA pretreated column. Fractions containing product Rf = 0.5 were pooled and evaporated to an oil. The oil was suspended in acetonitrile, filtered with a 0.45um syringe filter, evaporated to a foam, dissolved in benzene and lyophilized to give 3.98g colorless lyophylate at 71%.

### 2'OTBS Phosphine SPTE protected nucleosides

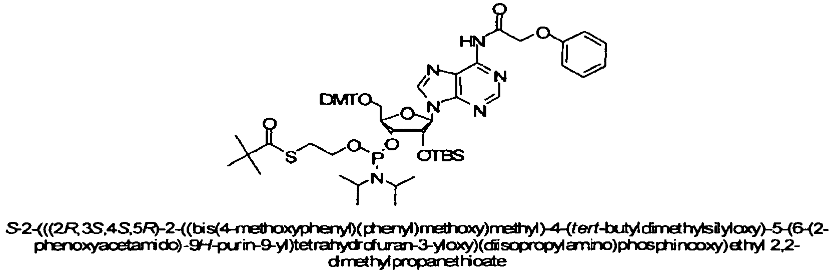

**VI_199:** Protected nucleoside(2.33g, 2.85mmol) and hydrotetrazolide (488mg, 2.85mmol) were diluted with DCM. To the stirring solution was added the phosphorodiamidite (1.18g, 3.0mmol) and the reaction was allowed to proceed for 30m. The reaction was quenched by pouring the crude into a saturated solution of sodium bicarbonate and washing with brine washed 3X10ml. The organic was dried over NaSO4 and evaporated. Isolation was achieved by chromatography DCM:EthylAcetate:TEA 45:45:10. Fractions containing product were evaporated followed by transfer/filtration through a 0.2um filter in benzene and lyophilization from benzene overnight to ensure complete removal of water. 2.1g lyophilate was recovered at 67%. ³¹P NMR (162 MHz, *d₆Benzene*) δ ppm 150.34, 148.79.

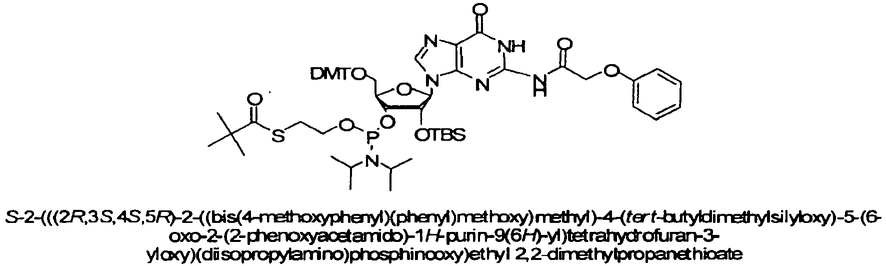

**VI_201:** Protected nucleoside(2.38g, 2.85mmol) and hydrotetrazolide (448mg, 2.85mmol) were diluted with DCM. To the stirring solution was added the phosphorodiamidite VI_253 (1.18g, 3mmol) and the reaction was allowed to proceed for 30m. The reaction was quenched by pouring the crude into a saturated solution of sodium bicarbonate and washing with brine washed 3X10ml. The organic was dried over NaSO4 and evaporated. Isolation was achieved by chromatography DCM:EthylAcetate:TEA 45:45:10. Fractions containing product were evaporated followed by transfer/filtration through a 0.45um filter in benzene and lyophilization from benzene overnight to ensure complete removal of water. Recovered 815mg lyophilate recovered at 25.4%. ³¹P NMR (162 MHz, *d₆Benzene*) δ ppm 149.22, 149.15.

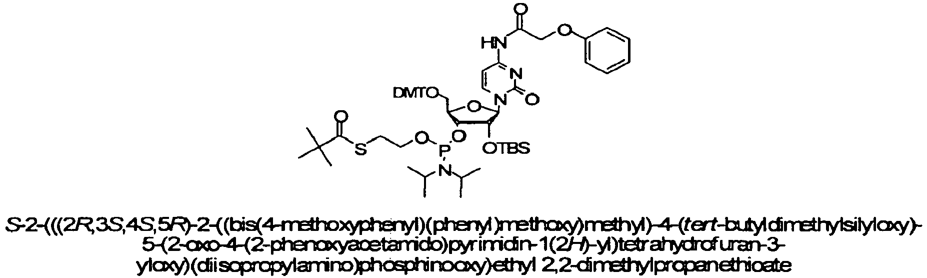

**VI_207:** Protected cytosine (3.77g, 4.75mmol) and SPTE phosphordiamidite VI_253 (1.96g, 5.0mmol) were dissolved in 19ml dichloromethane. Ethylthiotetrazole 0.45M in acetonitrile (19ml) was added and the reaction was found to be complete in 2.5h. Chromatography and filtration resulted in 4.3glyophilate at 83.4%. ³¹P NMR (162 MHz, *d₆Benzene*) δ ppm 150.5, 147.6.

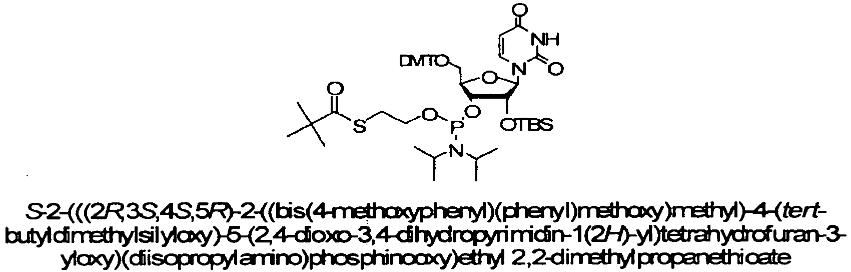

**VI_205:** Protected uridine (3.42g, 5.18mmol) and SPTE phosphordiamidite VI_253 (2.139g, 545mmol) were dissolved in 20.7ml dichloromethane. Ethylthiotetrazole 0.45M in acetonitrile (20.7) was added and the reaction was found to be complete in 2.5h. Chromatography and filtration resulted in 4.7g lyophilate at 95%. ³¹P NMR (162 MHz, *d₆Benzene*) δ ppm 149.9, 149.1.

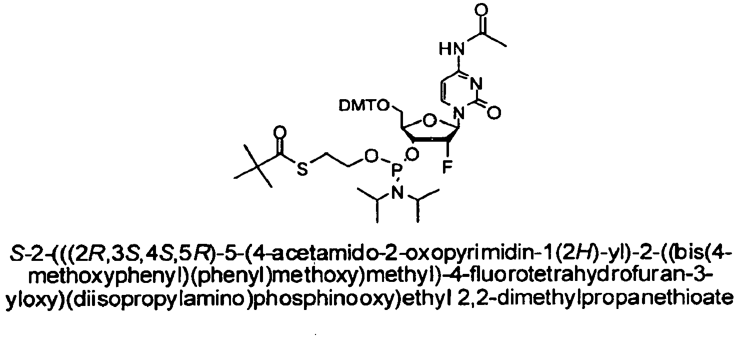

### 2'F nucleosides containing SPTE phosphine protecting groups:

**VI_239:** Protected cytosine (2.0g, 3.39mmol) and SPTE phosphordiamidite (14.02g, 3.57mmol) were dissolved in 13.6ml dichloromethane. Ethylthiotetrazole 0.45M in acetonitrile (13.6ml) was added and the reaction was found to be complete in 2.5h. Chromatography and filtration resulted in 2.22g lyophilate at 74%. ³¹P NMR (162 MHz, *d₆Benzene*) δ ppm 150.3, 150.2 ppm.

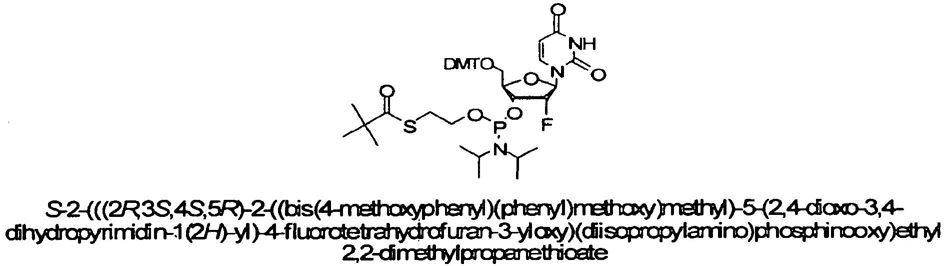

**VI_249:** Protected uridine (2.08g, 3.9mmol) and SPTE phosphordiamidite (15.7g, 4.0mmol) were dissolved in 15.2ml dichloromethane. Ethylthiotetrazole 0.45M in acetonitrile (15.2ml) was added and the reaction was found to be complete in 2.5h. Chromatography and filtration resulted in 2.6g lyophilate at 82%.

### 2'OMe modified oligonucleosides containing an SPTE phosphate protecting group

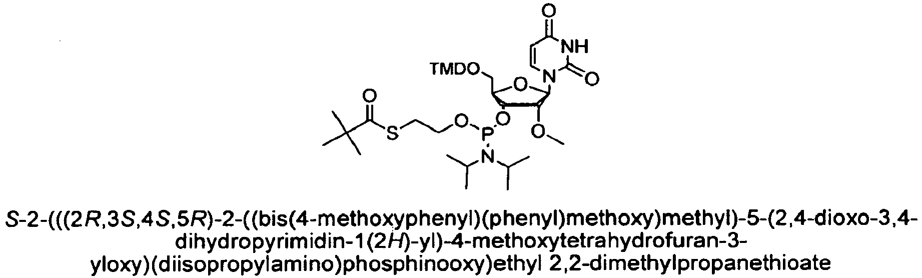

**VI_243:** 5'DMT 2'Omethyl uridine (lg, 1.8mmol) and Ethylthioltetrazole 0.45M (7.1ml) were added to a flame dried vial equipped with a stir bar. To the stirring solution was added 7ml dichloromethane and the phosphorodiamidite VI_253 (738.1mg, 1.88mmol) and the reaction was allowed to proceed for 3h. The reaction concentrated to a viscous oil and Isolation was achieved by TEA pretreated silica chromatography. Fractions containing product were evaporated followed by transfer/filtration through a 0.45um filter in acetonitrile and lyophilized from benzene overnight to ensure complete removal of water and residual triethylamine. 1.3g of lyophilate was recovered at 85.4%.

### Alternate post oligonucleotide synthesis routes to phosphotriesters:

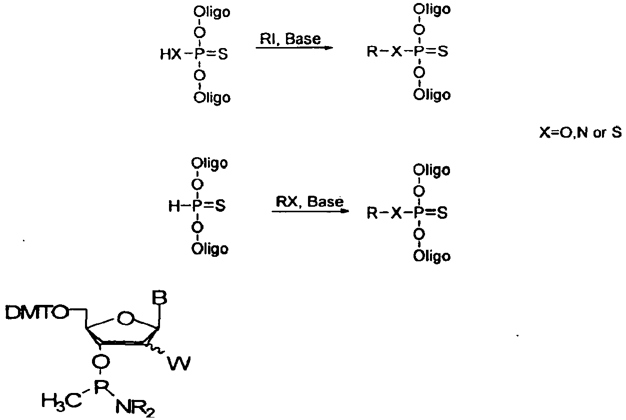

Methylphosphonates of the general structure listed above also provide access to oligonucleotides with charge neutral backbones that additionally provide nuclease resistance.

The examples set forth above are provided to give those of ordinary skill in the art a complete disclosure and description of how to make and use the embodiments of the apparatus, systems and methods of the disclosure, and are not intended to limit the scope of what the inventors regard as their disclosure.

## Claims

1. A pharmaceutical composition comprising a nucleic acid construct comprising:
a double-stranded oligonucleotide or polynucleotide domain comprising a phosphodiester and/or phosphothioate protecting group at one or more positions that reduces the net anionic charge of the double-stranded oligonucleotide or polynucleotide backbone, wherein the phosphodiester and/or phosphothioate protecting group combines with the phosphodiester or phosphothioate to form a group having the general formula: 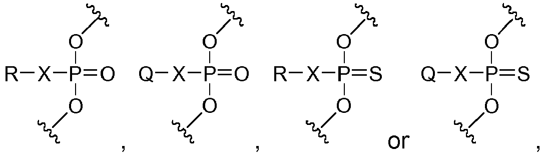 and wherein the double-stranded oligonucleotide or polynucleotide comprises a 2'-**W** moiety, wherein **W** is F, or O-alkyl;
wherein X is O or S or NR¹;
wherein R¹ is H, methyl, ethyl, S-pivaloyl thioethanol, hydroxyl, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic or substituted heterocyclic;
wherein R is selected from the group consisting of:
(i) R²,
wherein R² is alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic or substituted heterocyclic;
(ii) 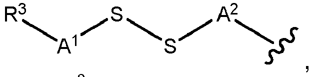
wherein R³ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
wherein A¹ and A² are each independently one to seven atom chains, or substituted one to seven atom chains;
(iii) 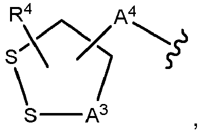
wherein R⁴ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
wherein A³ and A⁴ are each independently one to seven atom chains, or substituted one to seven atom chains;
(iv) 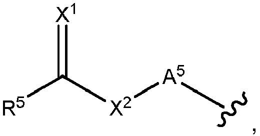
wherein R⁵ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic, halo, cyano or nitro,
wherein A⁵ is a one to seven atom chain, or substituted one to seven atom chain,
wherein X¹ is O, S or NR⁷, and wherein X² is O, S, or NR⁷, and R⁷ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic; and
(v) 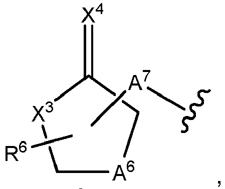
wherein R⁶ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, substituted heterocyclic, halo, cyano, or nitro,
wherein A⁶ and A⁶ are each independently one to seven atom chains, or substituted one to seven atom chains,
wherein X³ is O, S or NR⁸, and wherein X⁴ is O, S or NR⁸, and R⁸ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic;
wherein Q is selected from the group consisting of:
(i) Q¹,
wherein Q¹ is a basic group with a pKa greater than or equal to 10,
(ii) 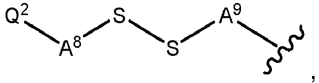
wherein Q² is a basic group with a pKa greater than or equal to 10,
wherein A⁸ and A⁹ are each independently one to seven atom chains, or substituted one to seven atom chains;
(iii) 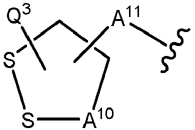
wherein Q³ is a basic group with a pKa greater than or equal to 10,
wherein A¹⁰ and A¹¹ are each independently one to seven atom chains, or substituted one to seven atom chains;
(iv) 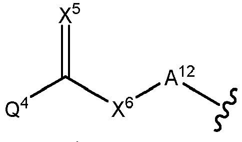
wherein Q⁴ is a basic group with a pKa greater than or equal to 10,
wherein A¹² is a one to seven atom chain, or substituted one to seven atom chain,
wherein X⁵ is O, S or NR⁹, and wherein X⁶ is O, S or NR⁹, and R⁹ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic; and
(v) 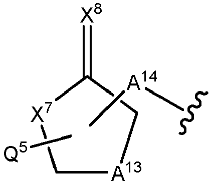
wherein Q⁵ is a basic group with a pKa greater than or equal to 10,
wherein A¹³ and A¹⁴ are each independently one to seven atom chains, or substituted one to seven atom chains,
wherein X⁷ is O, S or NR¹⁰, and wherein X⁸ is O, S or NR¹⁰, and R¹⁰ is H, hydroxy, alkyl, substituted alkyl, alkoxy, substituted alkoxy, cycloalkyl, substituted cycloalkyl, alkenyl, substituted alkenyl, alkynyl, substituted alkynyl, aryl, substituted aryl, heterocyclic, or substituted heterocyclic.

2. The pharmaceutical composition of claim 1, wherein **W** is F or O-Me.

3. The pharmaceutical composition of claim 1 or 2 further comprising at least one transduction domain comprising a membrane transport function operably linked to the oligonucleotide or polynucleotide domain.

4. The pharmaceutical composition of claim 3, wherein said at least one transduction domain is a targeting domain.

5. The pharmaceutical composition of claim 4, wherein said targeting domain is a receptor, receptor ligand, or antibody, and said targeting domain binds a cognate binding domain expressed on a cell.

6. The pharmaceutical composition of claim 3, wherein said at least one transduction domain is a protein transduction domain.

7. The pharmaceutical composition of claim 6, wherein overall charge of the nucleic acid construct is neutral or positively charged relative to a non-protected oligonucleotide or polynucleotide.

8. The pharmaceutical composition of claim 6, wherein the at least one protein transduction domain is selected from the group consisting of a polypeptide comprising a herpesviral VP22 domain; a polypeptide comprising a human immunodeficiency virus (HIV) TAT domain; a polypeptide comprising a homeodomain of an Antennapedia protein (Antp HD) domain; an N-terminal cationic prion protein domain; poly-Arg; and functional fragments thereof.

9. The pharmaceutical composition of claim 1 or 2, wherein **R-X-** is MeO, EtO, iPrO, 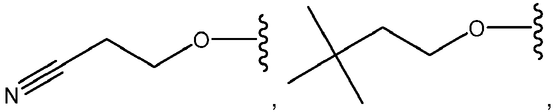 or

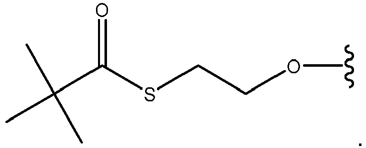

10. The pharmaceutical composition of claim 1 or 2, wherein phosphodiester and/or phosphothioate protecting group is a structure selected from the group consisting of: 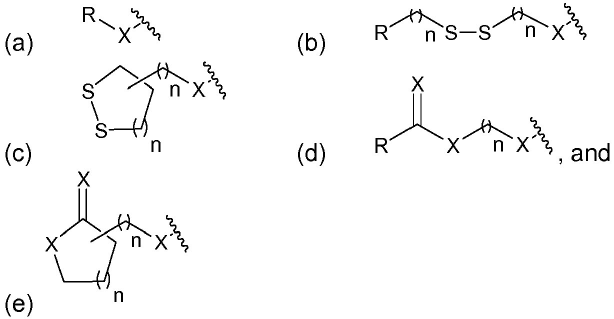
wherein: R = alkyl, heteroacyclic, aryl, cyclic, heterocyclic or H,
n = 1 to 7 atom linkage that can be alkyl, alkenyl, or alkyl heteroatom linkage combinations,
X = O, S, N.

11. The pharmaceutical composition of claim 1 or 2, wherein the nucleic acid construct comprises a basic substitution, guanidinium, amine, or urea group attached to the phosphodiester and/or phosphothioate protecting group via a linker to form an RNB protecting group.

12. The pharmaceutical composition of claim 11, wherein the RNB protecting group comprises a structure selected from the group consisting of: 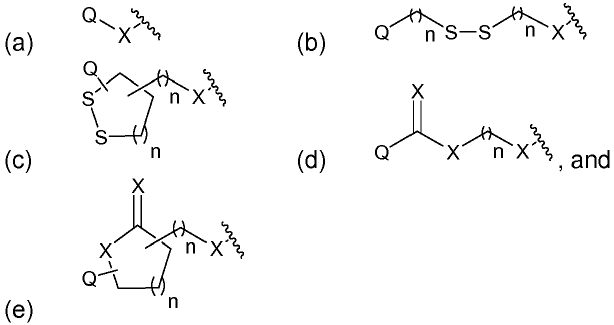 wherein:
n = 1 to 7 atom linkage that can be alkyl, alkenyl, or alkyl heteroatom linkage combinations,
X = O, S, N,
Q = basic substitution, guanidinium, amine, urea, attached via a linker.

13. A pharmaceutical composition of claim 1 or 2 for use in treating a disease or disorder, wherein oligonucleotide or polynucleotide comprises a therapeutic or diagnostic molecule.

14. The pharmaceutical composition of claim 3, wherein the at least one transduction domain is reversibly or irreversibly conjugated to the 5', 3' or both ends of the oligonucleotide or polynucleotide domain.

## Patentansprüche

1. Pharmazeutische Zusammensetzung, umfassend ein Nukleinsäurekonstrukt, das Folgendes umfasst:
eine doppelsträngige Oligonukleotid- oder Polynukleotid-Domäne, die eine Phosphodiester- und/oder Phosphothioat-Schutzgruppe an einer oder mehreren Positionen umfasst, durch die die anionische Nettoladung des doppelsträngigen Oligonukleotid- bzw. Polynukleotid-Grundgerüsts reduziert wird, wobei die Phosphodiester- und/oder Phosphothioat-Schutzgruppe zusammen mit dem Phosphodiester bzw. Phosphothioat eine Gruppe mit der allgemeinen Formel 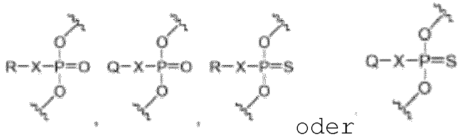 "umfasst und wobei das doppelsträngige Oligonukleotid oder Polynukleotid eine 2'-**W**-Gruppierung umfasst, wobei **W** für F oder O-Alkyl steht;
wobei X für O, S oder NR¹ steht;
wobei R¹ für H, Methyl, Ethyl, S-Pivaloylthioethanol, Hydroxyl, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl oder substituiertes Heterocyclyl steht;
wobei R ausgewählt ist aus der Gruppe bestehend aus:
(i) R²,
wobei R² für Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl oder substituiertes Heterocyclyl steht;
(ii) 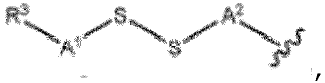 wobei R³ für H, Hydroxy, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl, substituiertes Heterocyclyl, Halogen, Cyano oder Nitro steht, wobei A¹ und A² jeweils unabhängig für Ketten mit einem bis sieben Atomen oder substituierte Ketten mit einem bis sieben Atomen stehen;
(iii) 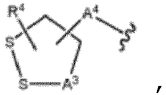 wobei R⁴ für H, Hydroxy, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl, substituiertes Heterocyclyl, Halogen, Cyano oder Nitro steht, wobei A³ und A⁴ jeweils unabhängig für Ketten mit einem bis sieben Atomen oder substituierte Ketten mit einem bis sieben Atomen stehen;
(iv) 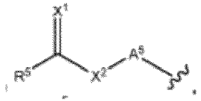
wobei R⁵ für H, Hydroxy, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl oder substituiertes Heterocyclyl, Halogen, Cyano oder Nitro steht,
wobei A⁵ für eine Kette mit einem bis sieben Atomen oder substituierte Kette mit einem bis sieben Atomen steht;
wobei X¹ für O, S oder NR⁷ steht und wobei X² für O, S oder NR⁷ steht und R⁷ für H, Hydroxy, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl oder substituiertes Heterocyclyl steht, und
(v) 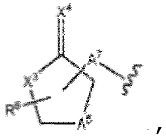
wobei R⁶ für H, Hydroxy, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl, substituiertes Heterocyclyl, Halogen, Cyano oder Nitro steht, wobei A⁶ und A⁶ jeweils unabhängig für Ketten mit einem bis sieben Atomen oder substituierte Ketten mit einem bis sieben Atomen stehen;
wobei X³ für O, S oder NR⁸ steht und wobei X⁴ für O, S oder NR⁸ steht und R⁸ für H, Hydroxy, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl oder substituiertes Heterocyclyl steht;
wobei Q ausgewählt ist aus der Gruppe bestehend aus:
(i) Q¹,
wobei Q¹ für eine basische Gruppe mit einem pKa-Wert größer oder gleich 10 steht,
(ii) 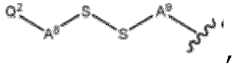
wobei Q² für eine basische Gruppe mit einem pKa-Wert größer oder gleich 10 steht,
wobei A⁸ und A⁹ jeweils unabhängig für Ketten mit einem bis sieben Atomen oder substituierte Ketten mit einem bis sieben Atomen stehen;
(iii) 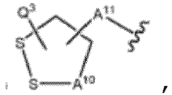
wobei Q³ für eine basische Gruppe mit einem pKa-Wert größer oder gleich 10 steht,
wobei A¹⁰ und A¹¹ jeweils unabhängig für Ketten mit einem bis sieben Atomen oder substituierte Ketten mit einem bis sieben Atomen stehen;
(iv) 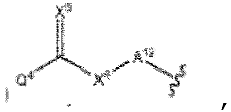
wobei Q⁴ für eine basische Gruppe mit einem pKa-Wert größer oder gleich 10 steht,
wobei A¹² für eine Kette mit einem bis sieben Atomen oder substituierte Kette mit einem bis sieben Atomen steht;
wobei X⁵ für O, S oder NR⁹ steht und wobei X⁶ für O, S oder NR⁹ steht und R⁹ für H, Hydroxy, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl oder substituiertes Heterocyclyl steht; und
(v) 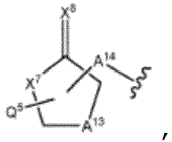
wobei Q⁵ für eine basische Gruppe mit einem pKa-Wert größer oder gleich 10 steht,
wobei A¹³ und A¹⁴ jeweils unabhängig für Ketten mit einem bis sieben Atomen oder substituierte Ketten mit einem bis sieben Atomen stehen;
wobei X⁷ für O, S oder NR¹⁰ steht und wobei X⁸ für O, S oder NR¹⁰ steht und R¹⁰ für H, Hydroxy, Alkyl, substituiertes Alkyl, Alkoxy, substituiertes Alkoxy, Cycloalkyl, substituiertes Cycloalkyl, Alkenyl, substituiertes Alkenyl, Alkinyl, substituiertes Alkinyl, Aryl, substituiertes Aryl, Heterocyclyl oder substituiertes Heterocyclyl steht.

2. Pharmazeutische Zusammensetzung nach Anspruch 1, wobei **W** für F oder O-Me steht.

3. Pharmazeutische Zusammensetzung nach Anspruch 1 oder 2, ferner umfassend wenigstens eine Transduktionsdomäne, die eine Membrantransportfunktion in operativer Verknüpfung mit der Oligonukleotid- oder Polynukleotid-Domäne umfasst.

4. Pharmazeutische Zusammensetzung nach Anspruch 3, wobei es sich bei der wenigstens einen Transduktionsdomäne um eine Targeting-Domäne handelt.

5. Pharmazeutische Zusammensetzung nach Anspruch 4, wobei es sich bei der Targeting-Domäne um einen Rezeptor, Rezeptorliganden oder Antikörper handelt und die Targeting-Domäne eine auf einer Zelle exprimierte zugehörige Bindungsdomäne bindet.

6. Pharmazeutische Zusammensetzung nach Anspruch 3, wobei es sich bei der wenigstens einen Transduktionsdomäne um eine Protein-Transduktionsdomäne handelt.

7. Pharmazeutische Zusammensetzung nach Anspruch 6, wobei die Gesamtladung des Nukleinsäurekonstrukts neutral oder positiv geladen relativ zu einem nicht geschützten Oligonukleotid oder Polynukleotid ist.

8. Pharmazeutische Zusammensetzung nach Anspruch 6, wobei die wenigstens eine Protein-Transduktionsdomäne aus der Gruppe bestehend aus einem Polypeptid, das eine Herpesvirus-VP22-Domäne umfasst; einem Polypeptid, das eine HIV(Human Immunodeficiency Virus)-TAT-Domäne umfasst; einem Polypeptid, das eine Homeodomäne einer Antennapedia-Protein(Antp HD)-Domäne umfasst; einer N-terminalen kationischen Prionprotein-Domäne; poly-Arg; und funktionsfähigen Fragmenten davon ausgewählt ist.

9. Pharmazeutische Zusammensetzung nach Anspruch 1 oder 2, wobei R-X- für MeO-, EtO-, iPrO, 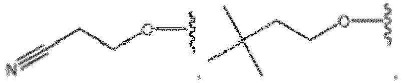 oder 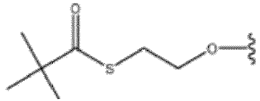 steht.

10. Pharmazeutische Zusammensetzung nach Anspruch 1 oder 2, wobei es sich bei der Phosphodiester- und/oder Phosphothioat-Schutzgruppe um eine Struktur handelt, die aus der Gruppe bestehend aus 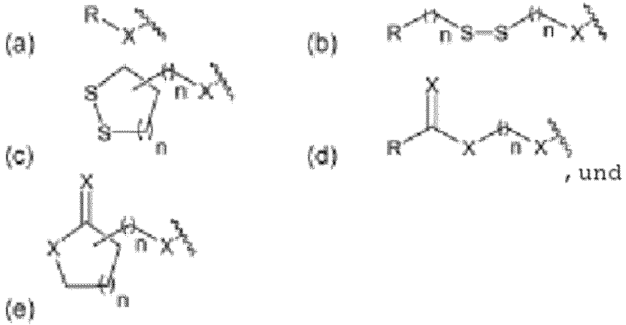
ausgewählt ist, wobei: R = Alkyl, Heteroacyclyl, Aryl, Cyclyl, Heterocyclyl oder H,
n = 1-bis-7-Atom-Verknüpfung, bei der es sich um Alkyl-, Alkenyl- oder Alkyl-Heteroatom-Verknüpfung-Kombinationen handeln kann,
X = O, S, N.

11. Pharmazeutische Zusammensetzung nach Anspruch 1 oder 2, wobei das Nukleinsäurekonstrukt eine basische Substitutions-, Guanidinium-, Amin- oder Harnstoffgruppe umfasst, die an die Phosphodiester- und/oder Phosphothioat-Schutzgruppe über einen Linker unter Bildung einer RNB-Schutzgruppe gebunden ist.

12. Pharmazeutische Zusammensetzung nach Anspruch 11, wobei die RNB-Schutzgruppe eine Struktur umfasst, die aus der Gruppe bestehend aus 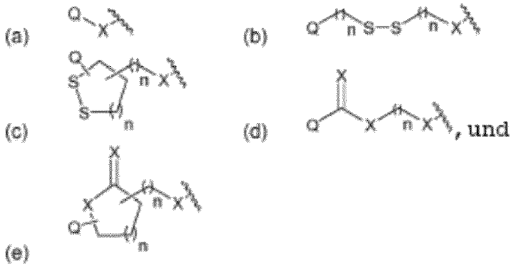 ausgewählt ist, wobei:
n = 1-bis-7-Atom-Verknüpfung, bei der es sich um Alkyl-, Alkenyl- oder Alkyl-Heteroatom-Verknüpfung-Kombinationen handeln kann,
X = O, S, N,
Q = basische Substitution, Guanidinium, Amin, Harnstoff, gebunden über einen Linker.

13. Pharmazeutische Zusammensetzung nach Anspruch 1 oder 2 zur Verwendung bei der Behandlung einer Krankheit oder Störung, wobei Oligonukleotid oder Polynukleotid ein therapeutisches oder diagnostisches Molekül umfasst.

14. Pharmazeutische Zusammensetzung nach Anspruch 3, wobei die wenigstens eine Transduktionsdomäne reversibel oder irreversibel an das 5'- oder/und 3'-Ende der Oligonukleotid- oder Polynukleotid-Domäne konjugiert ist.

## Revendications

1. Composition pharmaceutique comprenant une structure d'acide nucléique comprenant :
un domaine oligonucléotidique ou polynucléotidique à double brin comprenant un groupement protecteur de phosphodiester et/ou phosphothioate en une ou plusieurs positions qui réduit la charge anionique nette du squelette oligonucléotidique ou polynucléotidique à double brin, où le groupement protecteur de phosphodiester et/ou phosphothioate se combine au phosphodiester ou au phosphothioate pour former un groupement répondant à la formule générale : 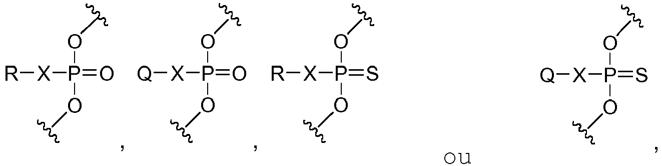 et où l'oligonucléotide ou polynucléotide à double brin comprend une entité 2'-W, où W représente F ou O-alkyle ;
où X représente O, S ou NR¹ ;
où R¹ représente H ou un groupement méthyle, éthyle, S-pivaloyle, thioéthanol, hydroxyle, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique ou hétérocyclique substitué ;
où R est choisi dans le groupe constitué par les suivants :
(i) R²,
où R² représente un groupement alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique ou hétérocyclique substitué ;
(ii) 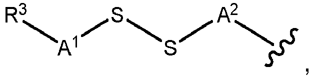
où R³ représente H ou un groupement hydroxy, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique, hétérocyclique substitué, halogéno, cyano ou nitro,
où chacun des chaînons A¹ et A² représente indépendamment une chaîne constituée d'un à sept atomes, ou une chaîne substituée constituée d'un à sept atomes ;
(iii) 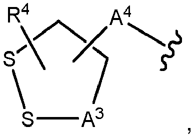
où R⁴ représente H ou un groupement hydroxy, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique, hétérocyclique substitué, halogéno, cyano ou nitro,
où chacun des chaînons A³ et A⁴ représente indépendamment une chaîne constituée d'un à sept atomes, ou une chaîne substituée constituée d'un à sept atomes ;
(iv) 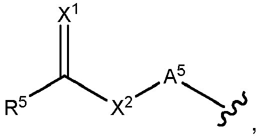
où R⁵ représente H ou un groupement hydroxy, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique ou hétérocyclique substitué, halogéno, cyano ou nitro,
où A⁵ représente indépendamment une chaîne constituée d'un à sept atomes, ou une chaîne substituée constituée d'un à sept atomes ;
où X¹ représente O, S ou NR⁷, et où X² représente O, S ou NR⁷, et où R⁷ représente H ou un groupement hydroxy, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique ou hétérocyclique substitué ; et
(v) 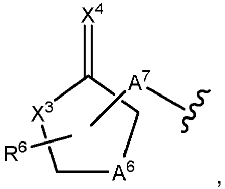
où R⁶ représente H ou un groupement hydroxy, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique, hétérocyclique substitué, halogéno, cyano ou nitro,
où chacun des chaînons A⁶ et A⁷ représente indépendamment une chaîne constituée d'un à sept atomes, ou une chaîne substituée constituée d'un à sept atomes,
où X³ représente O, S ou NR⁸, et où X⁴ représente O, S ou NR⁸, et où R⁸ représente H ou un groupement hydroxy, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique ou hétérocyclique substitué ;
où Q est choisi dans le groupe constitué par les suivants :
(i) Q¹,
où Q¹ représente un groupement basique de pKa supérieur ou égal à 10,
(ii) 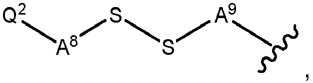
où Q² représente un groupement basique de pKa supérieur ou égal à 10,
où chacun des chaînons A⁸ et A⁹ représente indépendamment une chaîne constituée d'un à sept atomes, ou une chaîne substituée constituée d'un à sept atomes ;
(iii) 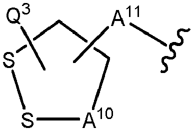
où Q³ représente un groupement basique de pKa supérieur ou égal à 10,
où chacun des chaînons A¹⁰ et A¹¹ représente indépendamment une chaîne constituée d'un à sept atomes, ou une chaîne substituée constituée d'un à sept atomes ;
(iv) 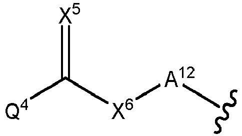
où Q⁴ représente un groupement basique de pKa supérieur ou égal à 10,
où A¹² représente indépendamment une chaîne constituée d'un à sept atomes, ou une chaîne substituée constituée d'un à sept atomes ;
où X⁵ représente O, S ou NR⁹, et où X⁶ représente O, S ou NR⁹, et où R⁹ représente H ou un groupement hydroxy, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique ou hétérocyclique substitué ; et
(v) 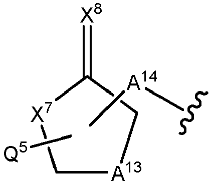
où Q⁵ représente un groupement basique de pKa supérieur ou égal à 10,
où chacun des chaînons A¹³ et A¹⁴ représente indépendamment une chaîne constituée d'un à sept atomes, ou une chaîne substituée constituée d'un à sept atomes,
où X⁷ représente O, S ou NR¹⁰, et où X⁸ représente O, S ou NR¹⁰, et où R¹⁰ représente H ou un groupement hydroxy, alkyle, alkyle substitué, alkoxy, alkoxy substitué, cycloalkyle, cycloalkyle substitué, alcényle, alcényle substitué, alcynyle, alcynyle substitué, aryle, aryle substitué, hétérocyclique ou hétérocyclique substitué.

2. Composition pharmaceutique selon la revendication 1, où W représente F ou O-Me.

3. Composition pharmaceutique selon la revendication 1 ou 2, comprenant en outre au moins un domaine de transduction comprenant une fonction de transport membranaire liée de façon fonctionnelle au domaine oligonucléotidique ou polynucléotidique.

4. Composition pharmaceutique selon la revendication 3, où ledit au moins un domaine de transduction est un domaine de ciblage.

5. Composition pharmaceutique selon la revendication 4, où ledit domaine de ciblage est un récepteur, ligand de récepteur ou anticorps, et ledit domaine de ciblage se lie à un domaine de liaison correspondant exprimé sur une cellule.

6. Composition pharmaceutique selon la revendication 3, où ledit au moins un domaine de transduction est un domaine de transduction de protéine.

7. Composition pharmaceutique selon la revendication 6, où la charge globale de la structure d'acide nucléique est neutre ou positive par rapport à un oligonucléotide ou polynucléotide non protégé.

8. Composition pharmaceutique selon la revendication 6, où l'au moins un domaine de transduction de protéines est choisi dans le groupe constitué par un polypeptide comprenant un domaine VP22 herpès viral ; un polypeptide comprenant un domaine TAT de virus d'immunodéficience humaine (VIH) ; un polypeptide comprenant un homéodomaine d'un domaine de protéine d'Antennapedia (Antp HD) ; un domaine de protéine de prion cationique N-terminal ; poly-Arg ; et leurs fragments fonctionnels.

9. Composition pharmaceutique selon la revendication 1 ou 2, où R-X- représente MeO, EtO, iPrO, 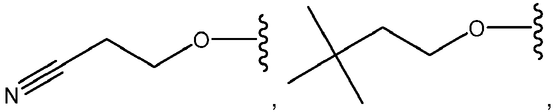 ou

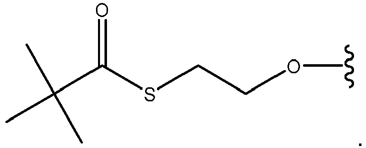

10. Composition pharmaceutique selon la revendication 1 ou 2, où le groupement protecteur phosphodiester et/ou phosphothioate est une structure choisie dans le groupe constitué par : 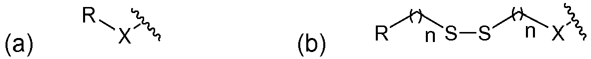 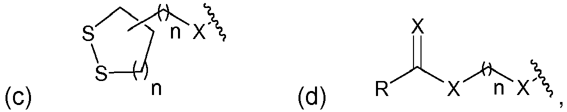 et 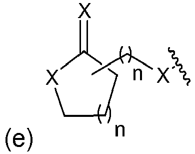
où : R = alkyle, hétérocyclique, aryle, cyclique, hétérocyclique ou H,
n = pont de 1 à 7 atomes qui peut être une combinaison de ponts alkyle, alcényle, ou alkyle-hétéroatomes,
X = O, S, N.

11. Composition pharmaceutique selon la revendication 1 ou 2, où la structure d'acide nucléique comprend un groupement de substitution basique, guanidinium, amine ou urée fixé au groupement protecteur de phosphodiester et/ou phosphothioate via un linker pour former un groupement protecteur RNB.

12. Composition pharmaceutique selon la revendication 11, où le groupement protecteur RNB comprend une structure choisie dans le groupe constitué par les suivants : 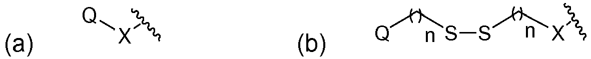 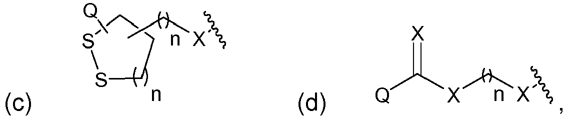 et 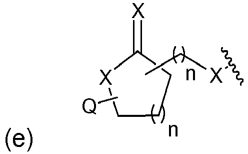 où :
n = pont de 1 à 7 atomes qui peut être une combinaison de ponts alkyle, alcényle, ou alkyle-hétéroatomes,
X = O, S, N,
Q = substitution basique, guanidinium, amine, urée, lié via un linker.

13. Composition pharmaceutique selon la revendication 1 ou 2 pour utilisation dans le traitement d'une maladie ou d'un trouble, où l'oligonucléotide ou le polynucléotide comprend une molécule thérapeutique ou diagnostique.

14. Composition pharmaceutique selon la revendication 3, où l'au moins un domaine de transduction est conjugué de façon réversible ou irréversible à l'extrémité 5', 3', ou aux deux, du domaine oligonucléotidique ou polynucléotidique.


### SEQUENCE LISTING

<110> The Regents of the University of California Dowdy, Steven F Petersen, Scott G Meade, Bryan R
<120> TRANSDUCIBLE DELIVERY OF NUCLEIC ACIDS BY REVERSIBLE PHOSPHOTRIESTER CHARGE NEUTRALIZATION PROTECTING GROUPS
<130> 00015-047WO1
<140> PCT/US07/15966
   <141> 2007-07-11
<150> US 60/830,572
   <151> 2006-07-12
<160> 24
<170> PatentIn version 3.4
<210> 1
   <211> 8
   <212> PRT
   <213> Artificial Sequence
<220>
   <223> Artificial Peptide consensus sequence
<220>
   <221> misc
   <222> (1)..(1)
   <223> X at position 1 is a basic amino acid
<220>
   <221> misc
   <222> (2)..(4)
   <223> X is any alpha-helix enhancing amino acid
<220>
   <221> misc
   <222> (5)..(5)
   <223> X at position 5 is a basic amino acid
<220>
   <221> misc
   <222> (6)..(7)
   <223> X is any alpha-helix enhancing amino acid
<220>
   <221> misc
   <222> (8)..(8)
   <223> X at position 8 is a basic amino acid
<400> 1
   Xaa Xaa Xaa Xaa Xaa Xaa Xaa Xaa
   1               5
<210> 2
   <211> 8
   <212> PRT
   <213> Artificial sequence
<220>
   <223> Artificial Peptide consensus sequence
<220>
   <221> misc
   <222> (1)..(1)
   <223> X at position 1 is a basic amino acid
<220>
   <221> misc
   <222> (2)..(3)
   <223> X is any alpha-helix enhancing amino acid
<220>
   <221> misc
   <222> (4)..(5)
   <223> X at position 4 and 5 are each independently a basic amino acid
<220>
   <221> misc
   <222> (6)..(7)
   <223> X is any alpha-helix enhancing amino acid
<220>
   <221> misc
   <222> (8)..(8)
   <223> X is a basic amino acid
<400> 2
   Xaa Xaa Xaa Xaa Xaa Xaa Xaa Xaa
   1               5
<210> 3
   <211> 7
   <212> PRT
   <213> Artificial sequence
<220>
   <223> Prion Protein Fragment
<400> 3
   Lys Lys Arg Pro Lys Pro Gly
   1               5
<210> 4
   <211> 11
   <212> PRT
   <213> Artificial sequence
<220>
   <223> Artificial Peptide consensus sequence
<220>
   <221> misc
   <222> (1)..(2)
   <223> X is any alpha-helix enhancing amino acid
<220>
   <221> misc
   <222> (4)..(4)
   <223> X is any alpha-helix enhancing amino acid
<220>
   <221> misc
   <222> (5)..(5)
   <223> X is any alpha-helix enhancing amino acid or a proline
<220>
   <221> misc
   <222> (6)..(7)
   <223> x is any alpha-helix enhancing amino acid or a basic amino acid
<220>
   <221> misc
   <222> (8)..(8)
   <223> X is any alpha-helix enhancing amino acid or a proline
<220>
   <221> misc
   <222> (9)..(9)
   <223> X is any alpha-helix enhancing amino acid
<220>
   <221> misc
   <222> (10)..(11)
   <223> X is any alpha-helix enhancing amino acid or a basic amino acid
<400> 4
   Xaa Xaa Arg Xaa Xaa Xaa Xaa Xaa Xaa Xaa Xaa
   1               5                   10
<210> 5
   <211> 5
   <212> PRT
   <213> Artificial Sequence
<220>
   <223> Artificial Peptide consensus sequence
<220>
   <221> misc
   <222> (2)..(2)
   <223> X is arginine or lysine
<220>
   <221> misc
   <222> (4)..(4)
   <223> x is any amino acid
<220>
   <221> misc
   <222> (5)..(5)
   <223> X is an arginine or lysine
<400> 5
   Lys Xaa Arg Xaa Xaa
   1               5
<210> 6
   <211> 9
   <212> PRT
   <213> Artificial sequence
<220>
   <223> Synthesized cationic Peptide Sequence
<400> 6
   Arg Lys Lys Arg Arg Gln Arg Arg Arg
   1               5
<210> 7
   <211> 86
   <212> PRT
   <213> Human immunodeficiency virus type 1
<400> 7
   Met Glu Pro Val Asp Pro Arg Leu Glu Pro Trp Lys His Pro Gly Ser
   1               5                   10                  15
   Gln Pro Lys Thr Ala Cys Thr Asn Cys Tyr Cys Lys Lys Cys Cys Phe
               20                  25                  30
   His Cys Gln Val Cys Phe Ile Thr Lys Ala Leu Gly Ile Ser Tyr Gly
           35                  40                  45
   Arg Lys Lys Arg Arg Gln Arg Arg Arg Pro Pro Gln Gly Ser Gln Thr
       50                  55                  60
   His Gln Val Ser Leu Ser Lys Gln Pro Thr Ser Gln Ser Arg Gly Asp
   65                  70                  75                  80
   Pro Thr Gly Pro Lys Glu
                   85
<210> 8
   <211> 5
   <212> PRT
   <213> Artificial sequence
<220>
   <223> Peptide Linker Sequence
<400> 8
   Gly Gly Gly Gly Ser
   1               5
<210> 9
   <211> 5
   <212> PRT
   <213> Artificial Sequence
<220>
   <223> Peptide Linker Sequence
<220>
   <221> misc
   <222> (1)..(5)
   <223> GGGGS is repeated two or more times
<400> 9
   Gly Gly Gly Gly Ser
   1               5
<210> 10
   <211> 12
   <212> PRT
   <213> Artificial sequence
<220>
   <223> Peptide Linker Sequence
<400> 10
   Gly Lys Ser Ser Gly Ser Gly Ser Glu Ser Lys Ser
   1               5                   10
<210> 11
   <211> 14
   <212> PRT
   <213> Artificial Sequence
<220>
   <223> Peptide Linker Sequence
<400> 11
   Gly Ser Thr Ser Gly Ser Gly Lys Ser Ser Glu Gly Lys Gly
   1               5                   10
<210> 12
   <211> 18
   <212> PRT
   <213> Artificial sequence
<220>
   <223> Peptide Linker Sequence
<400> 12
   Gly Ser Thr Ser Gly Ser Gly Lys Ser Ser Glu Gly Ser Gly Ser Thr
   1               5                   10                  15
   Lys Gly
<210> 13
   <211> 18
   <212> PRT
   <213> Artificial sequence
<220>
   <223> Peptide Linker Sequence
<400> 13
   Gly Ser Thr Ser Gly Ser Gly Lys Pro Gly Ser Gly Glu Gly Ser Thr
   1               5                   10                  15
   Lys Gly
<210> 14
   <211> 14
   <212> PRT
   <213> Artificial sequence
<220>
   <223> Peptide Linker sequence
<400> 14
   Glu Gly Lys Ser Ser Gly Ser Gly Ser Glu Ser Lys Glu Phe
   1               5                   10
<210> 15
   <211> 11
   <212> PRT
   <213> Artificial sequence
<220>
   <223> Artificial peptide cationic domain
<400> 15
   Ala Gly Arg Lys Lys Arg Arg Gln Arg Arg Arg
   1               5                   10
<210> 16
   <211> 11
   <212> PRT
   <213> Artificial sequence
<220>
   <223> Artificial peptide cationic domain
<400> 16
   Tyr Ala Arg Lys Ala Arg Arg Gln Ala Arg Arg
   1               5                   10
<210> 17
   <211> 11
   <212> PRT
   <213> Artificial Sequence
<220>
   <223> Artificial peptide cationic domain
<400> 17
   Tyr Ala Arg Ala Ala Ala Arg Gln Ala Arg Ala
   1               5                   10
<210> 18
   <211> 11
   <212> PRT
   <213> Artificial Sequence
<220>
   <223> Artificial peptide cationic domain
<400> 18
   Tyr Ala Arg Ala Ala Arg Arg Ala Ala Arg Arg
   1               5                   10
<210> 19
   <211> 11
   <212> PRT
   <213> Artificial sequence
<220>
   <223> Artificial peptide cationic domain
<400> 19
   Tyr Ala Arg Ala Ala Arg Arg Ala Ala Arg Ala
   1               5                   10
<210> 20
   <211> 11
   <212> PRT
   <213> Artificial sequence
<220>
   <223> Artificial peptide cationic domain
<400> 20
   Tyr Ala Arg Arg Arg Arg Arg Arg Arg Arg Arg
   1               5                   10
<210> 21
   <211> 11
   <212> PRT
   <213> Artificial Sequence
<220>
   <223> Artificial peptide cationic domain
<400> 21
   Tyr Ala Ala Ala Arg Arg Arg Arg Arg Arg Arg
   1               5                   10
<210> 22
   <211> 20
   <212> RNA
   <213> Artificial Sequence
<220>
   <223> synthesized oligonucleotide
<400> 22
   uuuuuuuuuu uuuucccuuu 20
<210> 23
   <211> 21
   <212> DNA
   <213> Artificial sequence
<220>
   <223> synthesized oligonucleotide
<400> 23
   ccacuaccug agcacccagt t 21
<210> 24
   <211> 21
   <212> DNA
   <213> Artificial Sequence
<220>
   <223> synthesized oligonucleotide
<400> 24
   ctgggtgctc aggtagtggt t 21